(19) Europäisches Patentamt
European Patent Office
Office européen des brevets

(11) **EP 3 788 366 B1**

## (12) EUROPEAN PATENT SPECIFICATION

(45) Date of publication and mention of the grant of the patent:
**17.05.2023 Bulletin 2023/20**

(21) Application number: **19724679.6**

(22) Date of filing: **03.05.2019**

(51) International Patent Classification (IPC):
**G01N 33/487** (2006.01)

(52) Cooperative Patent Classification (CPC):
**G01N 33/48721**

(86) International application number:
**PCT/US2019/030665**

(87) International publication number:
**WO 2019/213571 (07.11.2019 Gazette 2019/45)**

(54) **IMPROVED MEMBRANES FOR NANOPORE SENSING APPLICATIONS**

VERBESSERTE MEMBRANEN FÜR DETEKTIONSANWENDUNGEN MIT NANOPOREN

MEMBRANES AMÉLIORÉES POUR DES APPLICATIONS DE DÉTECTION À L'AIDE DE NANOPORES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2018 US 201862666489 P**

(43) Date of publication of application:
**10.03.2021 Bulletin 2021/10**

(73) Proprietor: **The Trustees of Wheaton College**
**Wheaton, IL 60187 (US)**

(72) Inventors:
• **BURDEN, Daniel, L.**
  **Wheaton, IL 60187 (US)**
• **BURDEN, Lisa, M.**
  **Wheaton, IL 60187 (US)**

(74) Representative: **Potter Clarkson**
**Chapel Quarter**
**Mount Street**
**Nottingham NG1 6HQ (GB)**

(56) References cited:
**US-B2- 9 797 863**

• **N. MALMSTADT ET AL: "Long-Lived Planar Lipid Bilayer Membranes Anchored to an In Situ Polymerized Hydrogel", ADVANCED MATERIALS, vol. 20, no. 1, 7 January 2008 (2008-01-07), pages 84-89, XP055059553, ISSN: 0935-9648, DOI: 10.1002/adma.200700810**
• **KAORI SUGIHARA ET AL: "A Gigaseal Obtained with a Self- Assembled Long-Lifetime Lipid Bilayer on a Single Polyelectrolyte Multilayer-Filled Nanopore", ACS, AMERICAN CHEMICAL SOCIETY, US, vol. 4, no. 9, 1 January 2010 (2010-01-01) , pages 5047-5054, XP007918431, ISSN: 1936-0851, DOI: 10.1021/NN100773Q [retrieved on 2010-08-05]**

**Description**

**CROSS REFERENCE TO RELATED APPLICATIONS**

This application claims the priority benefit of U.S. Provisional Patent Application No. 62/666,489, filed May 3, 2018.

**STATEMENT OF GOVERNMENT INTEREST**

This invention was made with government support under NSF 0550005 awarded by the National Science Foundation. The government has certain rights in the invention.

**FIELD OF THE INVENTION**

The present disclosure relates to membranes having enhanced stability and durability, methods of making the same, and uses thereof. Membranes of the disclosure are useful in technologies that depend on a lipid bilayer for success, e.g., nanopore sensing applications.

**BACKGROUND**

Over the past two decades, nanopore sensing applications have expanded rapidly [Howorka et al., Chemical Society reviews 2009, 38, 2360-2384; Kasianowicz et al., Biochimica et. biophysica acta 2016, 1858, 593-606; Kocer et al., Biosensors & bioelectronics 2012, 38, 1-10; Kudr et al., Electrophoresis 2015, 36, 2367-2379; Liu et al., International ed. in. English 2016, 55, 15216-15222; Liu et al., Mikrochimica acta 2016, 183, 955-963; Oukhaled et al., ACS chemical biology 2012, 7, 1935-1949; Schmidt, Current opinion in. biotechnology 2016, 39, 17-27; Shi et al., Analytical chemistry 2017, 89, 157-188]. Typically, these assays involve the creation of a single aqueous-filled pore of approximately 10° nm in an electrically impermeable barrier. Upon application of electrical potential, analytes are detected as they pass from one side of the pore to the other. A myriad of ions, small molecules, polymers, and nanoparticles can now be detected using biological nanopores, as well as pores milled in solid-state materials such as silicon nitride or graphene [Yang et al., Frontiers in. microbiology 2016, 7, 1500; Liu et al., Chemical Society reviews 2012, 41, 2283-2307]. More recently, assays have arisen that successfully extend the use of biological nanopores into complex chemical matrices, such as cell lysates and blood plasma [Fahie et al., Analytical chemistry 2015, 87, 11143-11149; Greninger et al., Genome medicine 2015, 7, 99; Gu et al., Nanopore Single-Molecule Detection of Circulating microRNAs. Clifton, N. J. 2013, 1024, 255-268; Kukwikila et al., Analytical chemistry 2015, 87, 9149-9154; Wang et al., ACS nano 2013, 7, 9814-9822; Wang et al., Nature nanotechnology 2011, 6, 668-674]. Together these factors suggest a bright future for nanopore sensing technologies, including commercial ventures aimed at a variety of markets that range from DNA sequencing to clinical microbiology.

Although the number and type of analyte species that are detectable with nanopores continues to grow, devices featuring protein nanopores have been limited, in part, by the membrane structures that incorporate the pore [Meier et al., Physical Chemistry Chemical Physics (Incorporating Faraday Transactions) 2000, 2, 4559-4562; Oshima et al., Analytical chemistry 2013, 85, 4363-4369; Hirano-Iwata et al., Langmuir: the ACS journal of surfaces and colloids 2010, 26, 1949-1952]. The earliest ion channel characterization platforms employed unsupported lipid films, often designated as black lipid membranes (BLMs), freely suspended across relatively large apertures (approximately $10^2$ $\mu$m diameter) [Miller, Ed.; In Ion Channel Reconstitution; Plenum Press: New York, 1986]. These lipid bilayers are ideal for protein nanopores owing to their fluidity, thickness (approximately 5 nm), organized layering of hydrophobic and hydrophilic regions in the membrane, highly resistive electrical properties (*e.g.*, Gohm seals). They also enable direct aqueous access to embedded nanopores from either side of the membrane. In essence, BLMs provide a simplistic approximation of a cell membrane, which is the native environment for ion channels. However, the mechanical fragility and short lifetimes of free-standing BLMs used for nanopore sensing applications creates time-consuming barriers to progress.

Various strategies have been developed to enhance the stability and mechanical properties of BLMs, including reducing aperture size [White et al., Journal of the American Chemical Society 2007, 129, 11766-11775; Baker et al., Analytical chemistry 2013, 85, 9078-9086], limiting the surface energy of the aperture substrates [Bright et al., ACS applied materials & interfaces 2013, 5, 11918-11926], forming bilayers on hydrated polymer cushions [Watkins et al., Langmuir: the ACS journal of surfaces and colloids 2011, 27, 13618-13628, see also Malmstadt et al., Advanced materials, 2008, 20, 84-89], tethering the bilayer to solid surfaces [Andersson, Tethered and Polymer Supported Bilayer Lipid Membranes: Structure and Function. 2016], trapping the bilayer between two hydrogel layers [Jeon et al., Journal of the American Chemical Society 2006, 128, 42-43], photopolymerizing reactive amphiphiles in the lipid membrane [Bright et al., ACS biomaterials science & engineering 2015, 1, 955-963], crosslinking of lipid molecules comprising the bilayer [Shenoy et al., Nano letters 2005, 5, 1181-1185], and the creation of bilayer structures using water droplets immersed in oil (known as droplet interface bilayers, or DIBs) [Heron et al., J. Am. Chem. Soc. 2007, 129, 16042-47]. Each approach has meritorious features, but limitations remain. For example, bilayers formed over a solid-supported polymer cushion provide greater tolerance to mechanical perturbation. But these structures have not yet been reported to give the electrical resistance needed for single-channel analysis [Kocer et al., Biosensors & bioelectronics 2012, 38, 1-10]. Similar challenges face strategies that tether the lipid bilayer to solid supports via covalent links to reactive sites on the solid surface [Kocer et al., Biosensors & bioelectronics 2012, 38, 1-10]. Membranes formed using photopolymerizable components possess enhanced mechanical rigidity. However, by nature, the lipid bilayer fluidity can be reduced, phase domains can interfere with bilayer stability [Shenoy et al., Nano letters 2005, 5, 1181-1185], and the conductivity of inserted pores can be altered [Bright et al., ACS biomaterials science & engineering 2015, 1, 955-963]. The hydrogel sandwich approach conveys notable durability and longevity; however, the thickness of the support structure reduces diffusive transport to the membrane surface by approximately 70% [Bright et al., ACS biomaterials science & engineering 2015, 1, 955-963].

## SUMMARY OF THE INVENTION

The invention is as set out in the claims, and provides:
A nanopore device comprising a membrane spanning an aperture, the membrane comprising:

a lipid bilayer that is linked to a filamentous network that is external to the membrane; and

at least one pore through the membrane; and

wherein the filamentous network is chemically linked to the lipid bilayer, the nanopore device characterized in that the filamentous network is a polypeptide, an oligonucleotide, or an oligosaccharide.

The invention also provides a method of analyzing a target polymer comprising contacting the target polymer to the nanopore device of the invention to allow the target polymer to move with respect to the at least one pore to produce a signal, and monitoring the signal corresponding to the movement of the target polymer with respect to the pore, thereby analyzing the target polymer.

The invention also provides a method of forming a nanopore device, the method comprising:

providing a membrane having a thickness and spanning an aperture, said membrane comprising a lipid bilayer;

associating the membrane with at least one pore such that the at least one pore extends through the membrane over the thickness of the membrane, thus forming at least one channel connecting a first side and a second side of the membrane, wherein the pore has a first opening that opens to the first side of the membrane, a second opening that opens to the second side of the membrane, and a depth; and
associating the membrane comprising at least one pore with a filamentous network that links to the first side of the membrane and is external to the membrane, thus forming the nanopore device, wherein the filamentous network is chemically linked to the membrane,

the method characterized in that the filamentous network is a polypeptide, an oligonucleotide, or an oligosaccharide.

Disclosed herein are materials and methods related to nanopore sensing application. Accordingly, the disclosure provides a nanopore device comprising a membrane spanning an aperture, the membrane comprising: a lipid bilayer that is linked to a filamentous network that is external to the membrane; and at least one pore through the membrane, wherein the filamentous network is a polypeptide, an oligonucleotide, or an oligosaccharide. In some embodiments, the nanopore device comprises at least one ion channel forming the at least one pore through the membrane. In further embodiments, the nanopore device comprises a plurality of apertures. In some embodiments, the device comprises one pore per aperture. In still further embodiments, the nanopore device comprises from about 1 to about 1,000,000 apertures. In further embodiments the nanopore device comprises about 1, or about 10, or about 20, or about 30, or about 40, or about 50, or about 60, or about 70, or about 80, or about 90, or about 100, or about 200, or about 500, or about 1,000, or about 2,000, or about 3,000, or about 5,000, or about 7,000, or about 10,000 apertures, or about 50,000, or about 100,000, or about 200,000, or about 300,000, or about 400,000, or about 500,000, or about 600,000, or about 700,000, or about 800,000, or about 900,000, or about 1,000,000 or more apertures.
In some embodiments, the ion channel is a protein ion channel, Staphylococcus aureus alpha-hemolysin, Bacillus anthracis protective antigen 63, gramicidin, MspA (Mycobacterium smegmatis), OmpF porin, Kapton, OmpG, ClyA (Salmonella typhimurium), a non-naturally occurring compound, or derivatives thereof.

The filamentous network of the device of the invention is a polypeptide, an oligonucleotide, or an oligosaccharide. The filamentous network is chemically linked to the lipid bilayer. In some embodiments, the chemical link is a covalent link. In further embodiments, the chemical link is non-covalent. In yet further embodiments, the non-covalent chemical link is a hydrogen bond, a high affinity complex (e.g., avidin/biotin), or a hydrophobic interaction. In some embodiments, the chemical link is ionic.

In some embodiments, the polypeptide is a cytoskeletal polypeptide. In further embodiments, the cytoskeletal polypeptide is linked to the lipid bilayer through a protein bridge.

In some embodiments, the aperture is about 10 nm to about 1 millimeter in diameter. In further embodiments, the aperture is about 50 microns to about 500 microns in diameter.

In some embodiments, the cytoskeletal polypeptide is a catenin, an intermediate filament protein, a microfilament protein, or a microtubule protein. In some embodiments, the catenin is alpha catenin, beta catenin, or gamma catenin. In further embodiments, the intermediate filament protein is desmin, glial fibrillary acidic protein, keratin, nestin, or vimentin. In some embodiments, the microfilament protein is actin, actinin, filamin, gelsolin, myosin, profilin, tensin, tropomyosin, troponin, or a derivative thereof. In some embodiments, the microtubule protein is dynein, tubulin (including alpha-, beta-, and gamma-tubulin), or kinesin. In some embodiments, the cytoskeletal protein is a homolog of actin, tubulin, or intermediate filaments. Such homologs arise, for example and without limitation, in bacteria and include molecules such as FtsZ, MreB, Mbl, ParM, and crescentin. In some embodiments, the polypeptide is a glycoprotein (e.g., laminin).

In some embodiments of the disclosure, the protein bridge comprises (i) biotin and streptavidin; (ii) spectrin; (iii) avidin, neutravidin, or a biotin binding protein; (iv) a bridge protein from the ERM family (e.g., ezrin, radixin, or moesin); (v) a bridge protein from the formin family (e.g., myosin I, integrin, tensin, catenin (alpha-, beta-, or gamma-); (vi) a transmembrane glycoprotein (e.g., CD44); or (iv) digoxygenin and an antibody directed against digoxygenin.

In further embodiments, a device of the disclosure further comprises a molecular motor, wherein said motor is adjacent to the at least one pore and is capable of moving a polymer with respect to the at least one pore. In some embodiments, the molecular motor comprises a DNA polymerase, a RNA polymerase, a ribosome, an exonuclease, or a helicase and said polymer is a polynucleotide. In further embodiments, the DNA polymerase is selected from E. coli DNA polymerase I, E. coli DNA polymerase I Large Fragment (Klenow fragment), phage T7 DNA polymerase, Phi-29 DNA polymerase, Thermus aquaticus (Taq) DNA polymerase, Thermus flavus (Tfl) DNA polymerase, Thermus Thermophilus (Tth) DNA polymerase, Thermococcus litoralis (Tli) DNA polymerase, Pyrococcus furiosus (Pfu) DNA polymerase, Bacillus stearothermophilus (Bst) DNA polymerase, AMV reverse transcriptase, MMLV reverse transcriptase, and HIV-1 reverse transcriptase. In some embodiments, the RNA polymerase is selected from T7 RNA polymerase, T3 RNA polymerase, SP6 RNA polymerase, and E. coli RNA polymerase. In still further embodiments, the exonuclease is selected from exonuclease Lambda, T7 Exonuclease, Exo III, RecJ1 Exonuclease, Exo I, and Exo T. In some embodiments, the helicase is selected from E-coli bacteriophage T7 gp4 and T4 gp41 gene proteins, E. coli protein DnaB, E. coli protein RuvB, and E. coli protein rho.

In some embodiments, the lipid bilayer comprises a plurality of lipid groups comprising one or more of diphytanoyl 1,2,-diacyl-sn-glycero-3-[phosphor-L-serine] (DiPHyPC), 1,2-dioleoyl-sn-glycero-3-phosphocholine (DOPC), 1,2-dimyristoyl-sn-phosphatidylcholine (DMPC), 1-palmitoyl-2-oleoyl-sn-phosphatidylcholine (POPC), 1,2-distearoyl-sn-glycero-3-phospho-(1'-rac-glycerol) (DSPG), 1,2-dioleoyl-sn-glycero-3-phospho-(1'-rac-glycerol) (DOPG), 1,2-distearoyl-sn-glycero-3-phosphocholine (DSPC), 1,2-dipalmitoyl-sn-glycero-3-phosphocholine (DPPC), 1,2-di-(9Z-octadecenoyl)-sn-glycero-3-phosphoethanolamine (DOPE), and 1,2-dihexadecanoyl-sn-glycero-3-phosphoethanolamine (DPPE).

In some aspects, the disclosure provides a method of analyzing a target polymer comprising contacting the target polymer to a nanopore device of the disclosure to allow the target polymer to move with respect to the at least one pore to produce a signal, and monitoring the signal corresponding to the movement of the target polymer with respect to the pore, thereby analyzing the target polymer. In some embodiments, the signal monitoring comprises measuring a monomer-dependent characteristic of the target polymer while the target polymer moves with respect to the pore. In further embodiments, the monomer dependent property is the identity of a monomer or the number of monomers in the polymer. In some embodiments, the method of analyzing a target polymer further comprises altering the rate of movement of the polymer before, during, or after the signal monitoring. In some embodiments, wherein the target polymer is an oligonucleotide, a polypeptide, or an oligosaccharide. In further embodiments, the oligonucleotide is DNA. In some embodiments, the analyzing comprises a chemical characterization. In some embodiments, the chemical characterization is a characterization of DNA, a synthetic polymer, a small molecule, or an ion. In further embodiments, the characterization of DNA comprises nucleotide sequencing or genotyping. In some embodiments, the analyzing reveals the identity of individual monomers of the target polymer. In further embodiments, the analyzing identifies the target polymer without measuring monomer-dependent characteristics (i.e., without monomer-level resolution).

In further aspects of the disclosure, a method of forming a nanopore device is provided, the method comprising: providing a membrane having a thickness and spanning an aperture, said membrane comprising a lipid bilayer; associating the membrane with at least one pore such that the at least one pore extends through the membrane over the thickness of the membrane, thus forming at least one channel connecting a first side and a second side of the membrane, wherein the pore has a first opening that opens to the first side of the membrane, a second opening that opens to the second side of the membrane, and a depth; and associating the membrane comprising at least one pore with a filamentous network that links to the first side of the membrane and is external to the membrane, thus forming the nanopore device. The filamentous network is a polypeptide, an oligonucleotide, or an oligosaccharide. The filamentous network is chemically linked to the membrane. In further embodiments, the chemical link is a covalent link. In some embodiments, the polypeptide is a cytoskeletal polypeptide. In further embodiments, the cytoskeletal polypeptide is linked to the membrane through a protein bridge. In some embodiments, the aperture is from about 10 nm to about 1 millimeter in diameter. In some embodiments, the aperture is from about 50 microns to about 500 microns in diameter. In further embodiments, the cytoskeletal polypeptide is a catenin, an intermediate filament protein, a microfilament protein, or a microtubule protein. In still further embodiments, the catenin is alpha catenin, beta catenin, or gamma catenin. In some embodiments, the intermediate filament protein is desmin, glial fibrillary acidic protein, keratin, nestin, or vimentin. In further embodiments, the microfilament protein is actin, actin-related protein, actinin, filamin, gelsolin, myosin, profilin, tensin, tropomyosin, or troponin. In some embodiments, the microtubule protein is dynein, tubulin, or kinesin. In further embodiments, the protein bridge comprises (i) biotin and streptavidin; (ii) spectrin; (iii) avidin, neutravidin, or a biotin binding protein; or (iv) digoxygenin and an antibody directed against digoxygenin. In some embodiments, the method further comprises a molecular motor, wherein said motor is adjacent to the at least one pore and is capable of moving a polymer with respect to the at least one pore. In some embodiments, the molecular motor comprises a DNA polymerase, a RNA polymerase, a ribosome, an exonuclease, or a helicase and said polymer is a polynucleotide. In further embodiments, the DNA polymerase is selected from E. coli DNA polymerase I, E. coli DNA polymerase I Large Fragment (Klenow fragment), phage T7 DNA polymerase, Phi-29 DNA polymerase, Thermus aquaticus (Taq) DNA polymerase, Thermus flavus (Tfl) DNA polymerase, Thermus Thermophilus (Tth) DNA polymerase, Thermococcus litoralis (Tli) DNA polymerase, Pyrococcus furiosus (Pfu) DNA polymerase, Bacillus stearothermophilus (Bst) DNA polymerase, AMV reverse transcriptase, MMLV reverse transcriptase, and HIV-1 reverse transcriptase. In some embodiments, the RNA polymerase is selected from T7 RNA polymerase, T3 RNA polymerase, SP6 RNA polymerase, and E. coli RNA polymerase. In some embodiments, the exonuclease is selected from exonuclease Lambda, T7 Exonuclease, Exo III, RecJ1 Exonuclease, Exo I, and Exo T. In further embodiments, the helicase is selected from E-coli bacteriophage T7 gp4 and T4 gp41 gene proteins, E. coli protein DnaB, E. coli protein RuvB, and E. coli protein rho. In some embodiments, the at least one pore is an ion channel. In some embodiments, the ion channel is a protein ion channel, Staphylococcus aureus alphahemolysin, Bacillus anthracis protective antigen 63, gramicidin, MspA (Mycobacterium smegmatis), OmpF porin, Kapton, OmpG, ClyA (Salmonella typhimurium), or a non-naturally occurring compound. In further embodiments, the lipid bilayer comprises a plurality of lipid groups comprising diphytanoyl 1,2,-diacyl-sn-glycero-3-[phosphor-L-serine] (DiPHyPC), 1,2-dioleoyl-sn-glycero-3-phosphocholine (DOPC), 1,2-dimyristoyl-sn-phosphatidylcholine (DMPC), 1-palmitoyl-2-oleoyl-sn-phosphatidylcholine (POPC), 1,2-distearoyl-sn-glycero-3-phospho-(1'-rac-glycerol) (DSPG), 1,2-dioleoyl-sn-glycero-3-phospho-(1'-rac-glycerol) (DOPG), 1,2-distearoyl-sn-glycero-3-phosphocholine (DSPC), 1,2-dipalmitoyl-sn-glycero-3-phosphocholine (DPPC), 1,2-di-(9Z-octadecenoyl)-sn-glycero-3-phosphoethanolamine (DOPE), or 1,2-dihexadecanoyl-sn-glycero-3-phosphoethanolamine (DPPE).

## BRIEF DESCRIPTION OF FIGURES

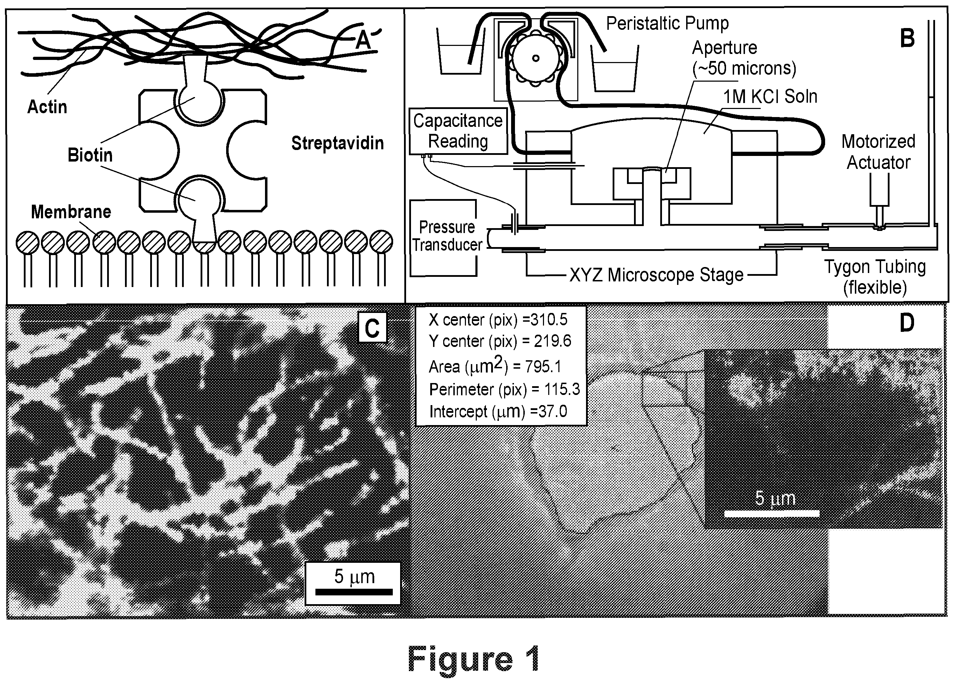

**Figure 1** shows (A) The f-actin network is chemically linked to the bilayer via a biotin-streptavidin-biotin bridge. (B) Schematic of the bilayer chamber used for simultaneous pressure control and measurement, membrane capacitance, channel conductance, and confocal optical microscopy. (C) f-actin filaments grew to be >10 $\mu$m in length using the preparation procedure described herein. Confocal image of a thin layer of fluorescently labeled filaments. (D) Brightfield image of a typical Teflon aperture. (Inset) Confocal fluorescent image acquired from this aperture with a lipid bilayer spanning the opening that is slightly deflected (*i.e.*, bulging) away from the confocal imaging plane. f-actin filaments are seen extending inwardly from the aperture rim.

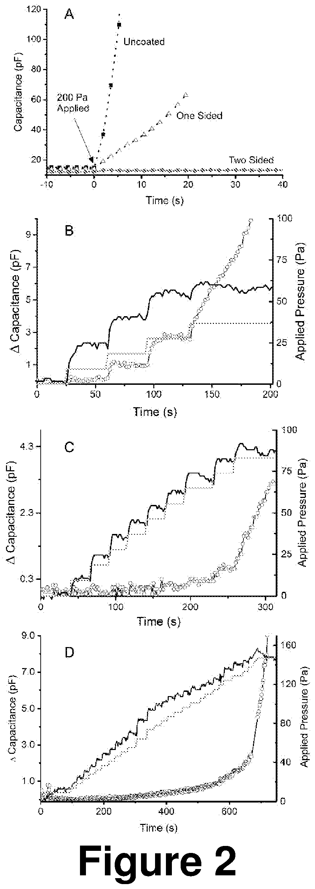

**Figure 2** shows (A) Single-step pressure test of bilayer membranes formed over the same aperture. At t=0, the trans bilayer pressure was raised to 200 Pa and the resulting membrane expansion was recorded. Shown in Figure 2 are the typical time courses for the expansion of an uncoated bilayer (squares), a bilayer with one side attached to an f-actin network (triangles), and a bilayer with both sides attached (gray circles) to an f-actin network. (B) Multistep pressure response of a typical uncoated bilayer. (C,D) Multi-step pressure response of a typical bilayer with one (C) and two (D) sides connected to an f-actin network (black: measured pressure; open circles: capacitance change, gray: unitless overlay of actuator position.

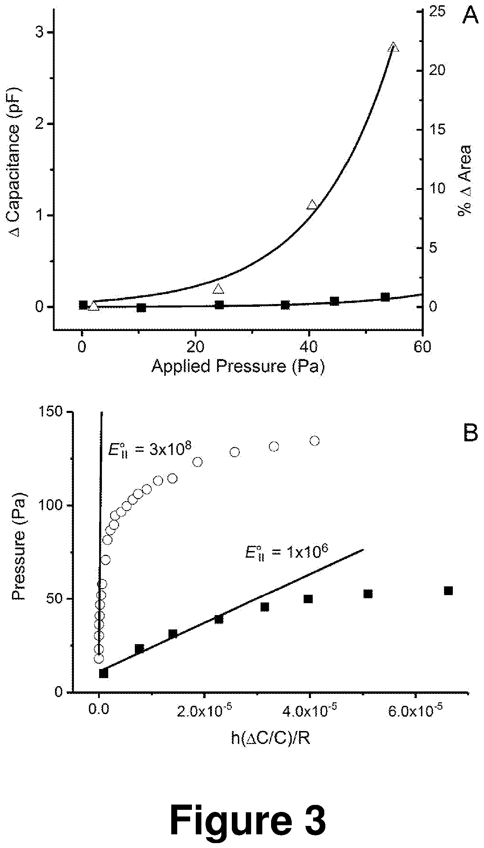

**Figure 3** shows (A) Comparison of the capacitance change and calibrated percent area increase upon trans-bilayer pressure application for an uncoated bilayer (open triangles) and a two-sided f-actin coated bilayer (closed squares). (B) Comparison of the initial elasticity modulus ($E_\parallel$) for uncoated (closed squares) and two-sided f-actin coated bilayer. f-actin coating conveyed a modulus increase of approximately 300x.

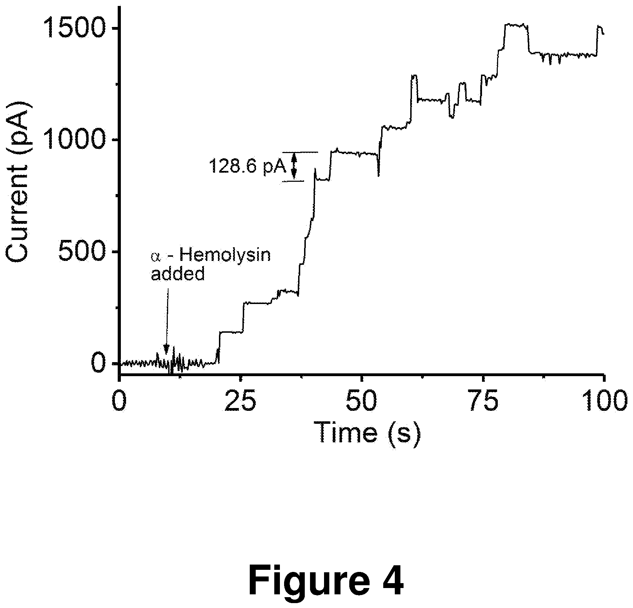

**Figure 4** shows results of an experiment in which ion channels were inserted into a bilayer coated with f-actin on both sides. Stepwise changes in current demonstrated that f-actin network anchored to the bilayer with a streptavidin/biotin linkage does not prohibit nominal nanopore activity.

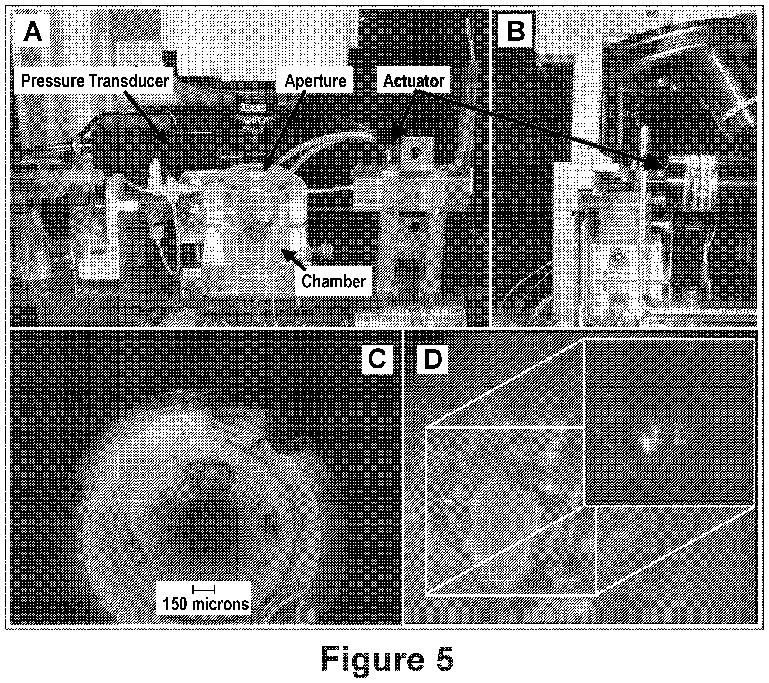

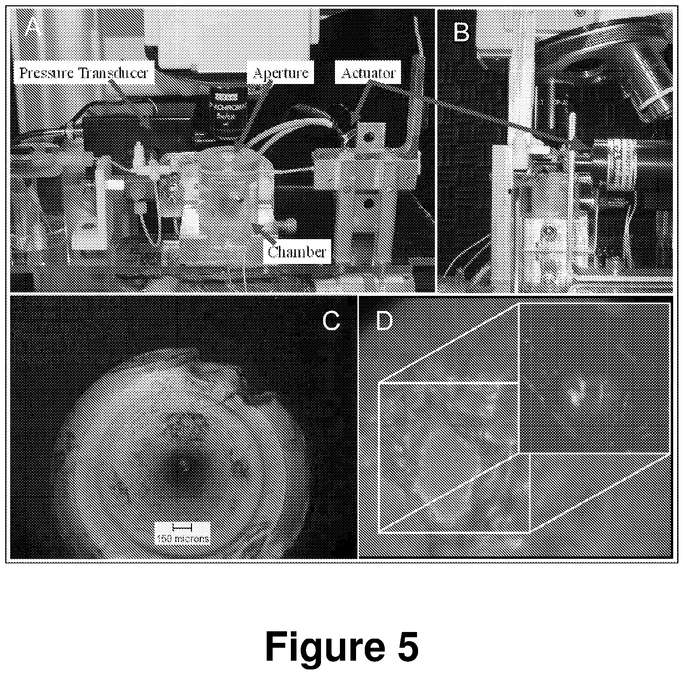

**Figure 5** shows the apparatus constructed to perform combined electrical and optical recordings on horizontally oriented lipid bilayers. **A** and **B** show the pressure control mechanism. **C** shows the top liquid-filled compartment and the surface of the Teflon plug illuminated from below. **D** shows combined bright-field and fluorescence images of an actin-coated membrane suspended across this aperture.

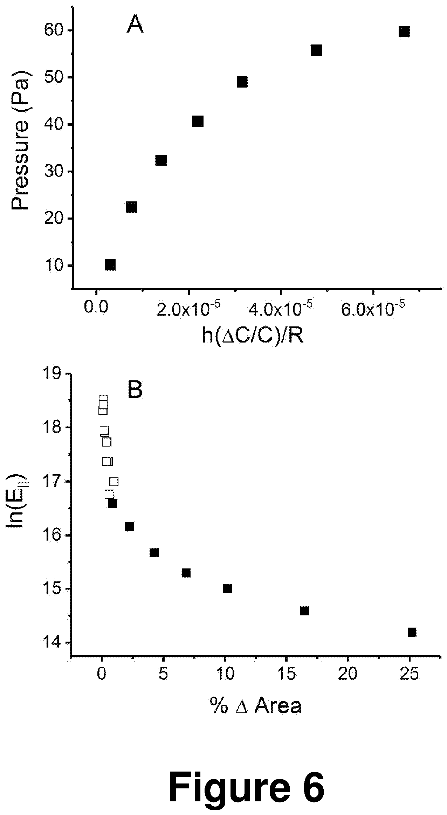

**Figure 6** shows the non-linearity of the in-plane elasticity modulus ($E_\parallel$) of an uncoated lipid bilayer as the area expansion grows large. **A** shows that $E_\parallel$ possesses its largest value when the pressure is low and the corresponding area expansion is small. **B** shows $E_\parallel$ values (closed squares) as a function of membrane surface expansion in comparison to similar measurements made by other investigators over smaller area expansions (open squares). The continuity of the two data sets shows that the absolute values of $E_\parallel$ are correct.

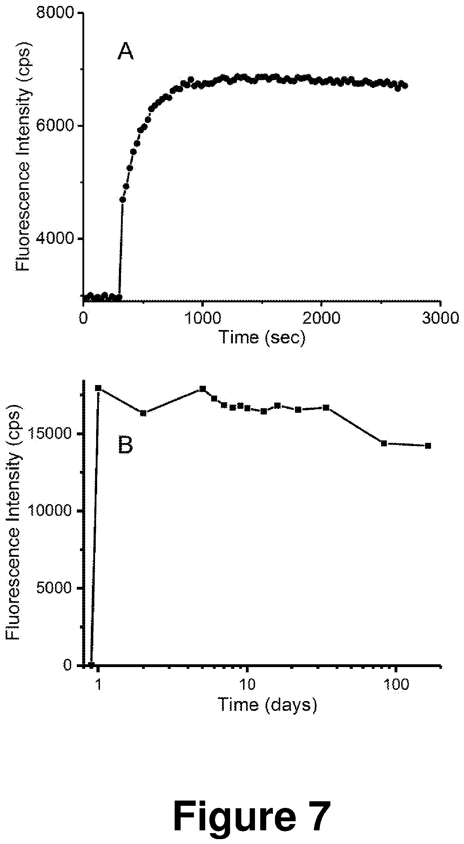

**Figure 7** shows f-actin polymerization kinetics and the resistance to degradation of bulk actin filaments. **A** shows that polymerization occurred rapidly after initiation by the addition of KCl, $MgCl_2$, and adenosine triphosphate. **B** shows fluorescence intensity from a bulk polymerized sample that was monitored over the course of many months.

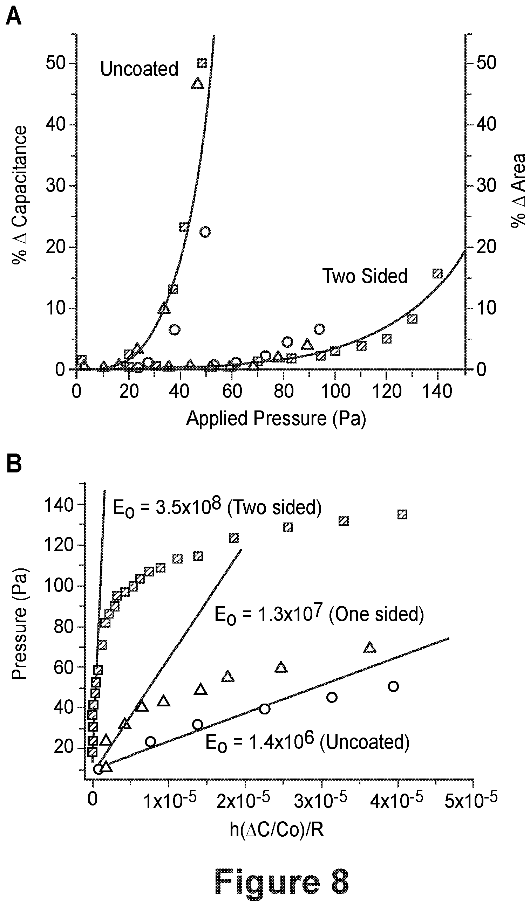

**Figure 8** shows (A) Comparison of the capacitance change and percent area increase upon trans-bilayer pressure application for an uncoated bilayer and a two-sided F-actin-coated bilayer. Replicate trials of each membrane type are shown with solid squares, open circles, and open triangles, respectively. Fits to each composite data set denote general trends. (B) Comparison of the average initial elastic moduli ($E_o$) for uncoated, one-sided, and two sided F-actin-coated bilayers. F-actin coating on two sides conveys a modulus increase of approximately $250\times$ when compared to uncoated bilayers. Data sets represent average moduli from 7, 6, and 3 trials (with standard deviations of $1.4 \times 10^6$, $9.4 \times 10^6$, and $3.0 \times 10^8$) for uncoated, one-sided, and two-sided membranes, respectively.

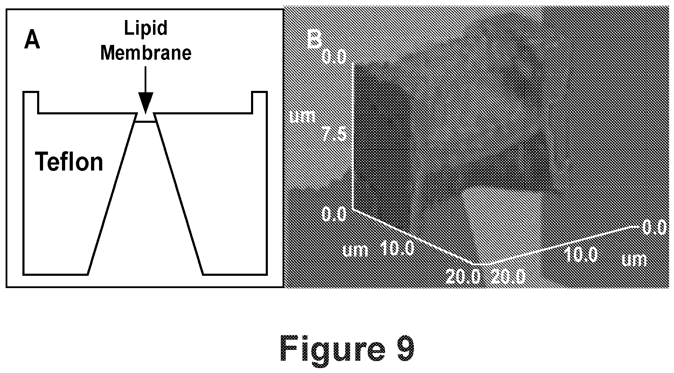

**Figure 9** shows (A) the typical contour of the tapered Teflon plug; and (B) a cut-away from a high-resolution confocal z stack collected at the end of the tapered Teflon plug.

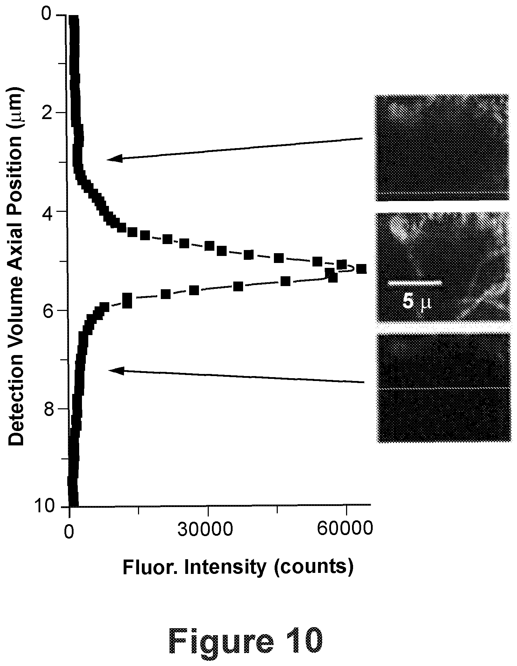

**Figure 10** shows a z stack of confocal images taken at 2-$\mu$m intervals from a two-sided f-actin-coated bilayer, as well as a high-resolution linear axial scan of the confocal detection volume through the coated bilayer.

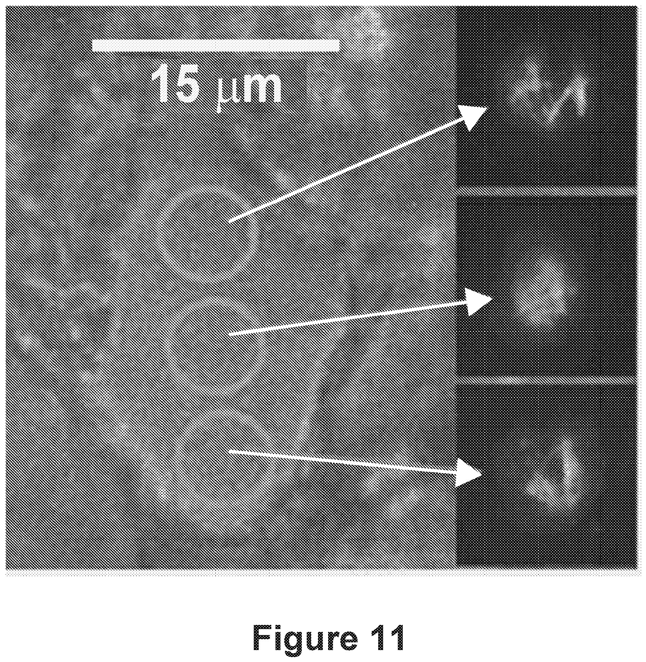

**Figure 11** shows widefield ICCD images from a two-sided f-actin-coated lipid bilayer where the illuminating laser beam has been expanded well beyond typical confocal diameters (*i.e.,* 7 $\mu$m diam.).

## DETAILED DESCRIPTION

All cells in all domains of life possess a cytoskeleton that provides mechanical resistance to deformation and general stability to the plasma membrane. The present disclosure provides a two-dimensional scaffolding created by an external filamentous network of molecules to convey mechanical support for relatively fragile planar bilayer membranes (Black Lipid Membranes, BLMs). In general, a BLM is a phospholipid bilayer that spans a small aperture. As provided herein, the membrane is a bilayer membrane. In further embodiments, the membrane is a droplet interface bilayer (DIB). The disclosure provides devices comprising (1) molecularly thin layers that are (2) linked to a membrane and applied in sequence to (3) build three-dimensional (3D) cross-linked networks of arbitrary thickness external to the membrane. The molecular-level thickness of the external filamentous network (*e.g.*, an actin filamentous network) maintains diffusive transport to the membrane surface, an advantage that a hydrogel does not provide.

More robust membranes are needed for the development of protein nanopore sensor applications. The devices disclosed herein mimic the manner in which spectrin anchors actin to cell membranes by implementing a biotin-streptavidin-biotin bridge to chemically link a web of polymerized actin to lipids in the membrane. The devices provided herein are characterized using various modes of optical microscopy, electrophysiology, and applied mechanical stress (including measurements of membrane elasticity). It is demonstrated herein that the resulting structure forms a thin, two-dimensional support that is stable over long periods of time (*e.g.*, days), with a support structure that remains intact for months.

Minimalistic layering of the support network provides elasticity enhancements of over 100-fold and increases with the number of applied layers. The device also retains lateral membrane fluidity, high electrical resistance, and uninhibited molecular access from bulk solution, which are highly desirable properties for nanopore sensing applications. Thus, the devices provided herein possess improved stability, longevity, durability, and resistance to mechanical stress relative to devices known in the art.

The term " membrane" as used herein is a layer that incorporates, in some embodiments, molecules of biological origin (*e.g.*, a lipid). A membrane of the disclosure is constructed from a non-natural amphiphile. A membrane can be a monolayer or a bilayer.

Unless otherwise defined herein, scientific and technical terms employed in the disclosure shall have the meanings that are commonly understood and used by one of ordinary skill in the art. Unless otherwise required by context, it will be understood that singular terms shall include plural forms of the same and plural terms shall include the singular. Specifically, as used herein and in the claims, the singular forms "a" and "an" include the plural reference unless the context clearly indicates otherwise.

## Methods of making a membrane

The design of the bilayer support disclosed herein closely mimics actual cytoskeletal structures that have produced success in living cells. A membrane according to the disclosure that is linked to an external filamentous network remains stable for extended periods of time, including indefinitely. Without such linkage to an external filamentous network, the membrane is much less stable and durable, and would quickly lose its structure. Like the hydrogel sandwich, the first step is to form a BLM that possesses electrical characteristics necessary for single-channel recording. Then, in a second step, a netting of molecular filaments (of approximate single-layer thickness or less) is linked to the bilayer. Filamentous networks that are external to the membrane are discussed further herein, and include, in some embodiments, filamentous actin. In some embodiments, chemical links to the membrane are formed using a bridge to connect the membrane to the filamentous network. In some embodiments, the bridge is a protein bridge. In further embodiments, the protein bridge comprises biotin and avidin which creates a strong integral connection to the hydrophobic interior. Accordingly, in some aspects, the disclosure provides a method of forming a nanopore device comprising providing a lipid bilayer having a thickness and spanning an aperture; providing at least one pore extending through the lipid bilayer over the thickness of the lipid bilayer, thus forming at least one channel connecting a first side and a second side of the lipid bilayer, wherein the pore has a first opening that opens to the first side of the lipid bilayer, a second opening that opens to the second side of the lipid bilayer, and a depth; providing a biomolecule that links to the first side of the lipid bilayer, thus forming the nanopore device.

In living cells, the formation and destruction of actin filaments (f-actin) is a continuously dynamic process that is driven by a host of molecular interactions [Hill et al., International review of cytology 1982, 78, 1-125]. Once stable filaments are formed, they can be anchored to the membrane by spectrin [Hartwig et al., Protein Profile 1994, 1, 706-778]. The unique interlocking structure of actin monomers forms filaments in a webbing that can propel shape changes in the plasma membrane. Yet, the actin web maintains a thickness that approximates a molecularly thin two-dimensional sheet with large openings. This system uniquely preserves several important membrane properties such as lipid fluidity, direct diffusional access to solution, and the high electrical resistance necessary for single-nanopore sensing applications.

Materials useful in the preparation of the devices of the present disclosure include those that serve as functional groups to create a lipid bilayer. For example and without limitation, the bilayer, in various embodiments, comprises a plurality of lipid functional groups comprising, for example and without limitation, diphytanoyl 1,2,-diacyl-sn-glycero-3-[phosphor-L-serine] (DiPHyPC), 1,2-dioleoyl-sn-glycero-3-phosphocholine (DOPC), 1,2-dimyristoyl-sn-phosphatidylcholine (DMPC), 1-palmitoyl-2-oleoyl-sn-phosphatidylcholine (POPC), 1,2-distearoyl-sn-glycero-3-phospho-(1'-rac-glycerol) (DSPG), 1,2-dioleoyl-sn-glycero-3-phospho-(1'-rac-glycerol) (DOPG), 1,2-distearoyl-sn-glycero-3-phosphocholine (DSPC), 1,2-dipalmitoyl-sn-glycero-3-phosphocholine (DPPC), 1,2-di-(9Z-octadecenoyl)-sn-glycero-3-phosphoethanolamine (DOPE), or 1,2-dihexadecanoyl-sn-glycero-3-phosphoethanolamine (DPPE). As a category, lipids are a group of natural and synthetic molecules that include fats/fatty acids, waxes, sterols, fat-soluble vitamins (such as vitamins A, D, E, and K), monoglycerides, diglycerides, triglycerides, and phospholipids. Each of these subcategories is contemplated by the disclosure. Thus, the disclosure contemplates any lipid compound may be used. Even more generally, any amphiphilic compound that can be used to create a membrane with hydrophilic and hydrophobic regions is contemplated to be useful in the devices and methods of the disclosure. Derivatized lipids are also contemplated by the disclosure. For example and without limitation, in some embodiments a lipid of the disclosure is biotinylated such that biotin is itself linked to the lipid head group using a carbonaceous tether ranging from a few to more than a dozen atoms.

In various embodiments, a device of the disclosure comprises polymerized lipids that span an aperture. In some embodiments, the lipid is a UV-polymerizable lipid (see, *e.g.*, U.S. Patent No. 8,294,007). Polymerizable lipids are an example of a kind of polymer network residing inside the membrane. The polymerizable lipid and/or polymerizable amphiphiles are internal to the membrane. According to the present disclosure, externally linked polymerizable networks have an advantage over internal polymer networks because they better sustain the high electrical resistance needed for single nanopore sensing measurements. Internally formed polymer networks provide mechanical support but can cause the bilayer to become electrically leaky. Externally linked filamentous networks as described herein allow the lipid to stay in its fluid form, maintaining good electrical insulation, while also adding structural support. In some embodiments, a device of the disclosure comprises a combination of externally linked polymer networks and internal polymerizable lipids.

In some embodiments, the pore of the device further comprises at least one ion channel forming at least one pore through the artificial membrane. Ion channels provide the molecular basis for nerve activity and mediate the selective transport of ions and macromolecules. In addition, some ion channels connect cells together to form large-scale functioning tissue whereas others act as lethal toxins. It has been shown that channels could act as components of sensors to detect a variety of analytes including ions, small molecules, polynucleotides, and polypeptides. According to further embodiments of the disclosure, the device comprises one pore per aperture. In some embodiments, the device comprises a plurality of apertures. In further embodiments, the device comprises from about 1 to about 1,000,000, or from about 1 to about 900,000, or from about 1 to about 800,000, or from 1 to about 700,000, or from about 1 to about 600,000, or from about 1 to about 500,000, or from about 1 to about 400,000, or from 1 to about 300,000, or from about 1 to about 200,000, or from about 1 to about 100,000, or from about 1 to about 50,000, or from about 1 to about 500, or from about 1 to about 50, or from about 2 to about 50, or from about 5 to about 50, or from about 10 to about 50, or from about 10 to about 40, or from about 10 to about 30, or from about 1 to about 1000, or from about 1 to about 10,000, or from about 10 to about 100, or from about 20 to about 50 apertures. In various embodiments, the device comprises about or at least about 10, about or at least about 20, about or at least about 30, about or at least about 40, about or at least about 50, about or at least about 60, about or at least about 70, about or at least about 80, about or at least about 90, about or at least about 100, about or at least about 200, about or at least about 500, about or at least about 1,000, about or at least about 2,000, about or at least about 3,000, about or at least about 5,000, about or at least about 7,000, about or at least about 10,000, about or at least about 50,000, about or at least about 100,000, about or at least about 200,000, about or at least about 300,000, about or at least about 400,000, about or at least about 500,000, about or at least about 600,000, about or at least about 700,000, about or at least about 800,000 about or at least about 900,000, about or at least about 1,000,000 or more apertures. In some embodiments, the aperture is from about 10 nanometers (nm) to about 1 millimeter (mm) in diameter. In further embodiments, the aperture is from about 10 nm to about 900 microns ($\mu$m), or from about 10 nm to about 800 $\mu$m, or from about 10 nm to about 700 $\mu$m, or from about 10 nm to about 600 $\mu$m, or from about 10 nm to about 500 $\mu$m, or from about 10 nm to about 400 $\mu$m, or from about 10 nm to about 300 $\mu$m, or from about 10 nm to about 200 $\mu$m, or from about 10 nm to about 100 $\mu$m, or from about 50 $\mu$m to about 500 $\mu$m, or from about 10 nm to about 1 $\mu$m in diameter. In further embodiments, the aperture is from about 10 $\mu$m to about 100 $\mu$m in diameter. In some embodiments, the aperture is about or at least about 10 nm, about or at least about 20 nm, about or at least about 30 nm, about or at least about 40 nm, about or at least about 50 nm, about or at least about 60 nm, about or at least about 70 nm, about or at least about 80 nm, about or at least about 90 nm, about or at least about 100 nm, about or at least about 200 nm, about or at least about 300 nm, about or at least about 400 nm, about or at least about 500 nm, about or at least about 600 nm, about or at least about 700 nm, about or at least about 800 nm, about or at least about 900 nm, about or at least about 1 $\mu$m, about or at least about 100 $\mu$m, about or at least about 200 $\mu$m, about or at least about 300 $\mu$m, about or at least about 400 $\mu$m, about or at least about 500 $\mu$m, about or at least about 600 $\mu$m, about or at least about 700 $\mu$m, about or at least about 800 $\mu$m, about or at least about 900 $\mu$m, or about or at least about 1 mm in diameter.

In further embodiments, a device of the disclosure is positioned over the top of a small well etched into a solid material. In some embodiments, the small well is a divot formed in glass by, for example and without limitation, micromachining or lithographic methods. Traditional apertures are understood in the art to comprise a small hole through a thin 2D film that separates two large aqueous-filled chambers. The volume of traditional chambers is on the order of milliliters. However, a device of the disclosure is contemplated to be implemented, in some embodiments, using an aperture created by a micromachined divot that is filled with a tiny amount of water (e.g., 10-1000 nanoliters). This type of aperture, which is defined by the rim of the micromachined divot, is effectively closed to bulk solution on the bottom side, but the top side of the aperture is in contact with a larger volume of water (see, e.g., U.S. Patent Application Publication No. 20150152494). Lipid membranes formed over well-type apertures can be more stable than traditional membranes formed on a 2D film partition. Use of a device of the disclosure will further improve stability and performance of membranes on well-type apertures.

The disclosure provides an "apertureless" membrane. Apertureless bilayer membranes are formed using droplet interface bilayer (DIB) technology (see, e.g., Holden et al. J. Am. Chem Soc. 129: 8650-8655 (2007); Leptihn et al., Nature Protocols 8: 1048-1-57 (2013); Tonooka et al., Small 10: 3275-3282 (2014); and Szabo et al., Biochim. Biophys. Acta 1858: 613-617 (2016)). This mode of forming a lipid bilayer involves placing aqueous droplets filled with a soluble amphiphile into a bath of oil. When two aqueous droplets are pressed together and make contact with each other, a bilayer membrane is formed. In some embodiments, a single aqueous droplet is forced into contact with a flat agarose surface (hydrogel) that has been coated with lipid to form a bilayer between the aqueous droplet interior and the coated hydrogel surface. The external filamentous network as described herein is contemplated for use on such a bilayer. Specifically, a filamentous network of the disclosure is created and chemically linked to the bilayer from the aqueous phase.

In some embodiments, the ion channel forms a pore all the way through the membrane. In some embodiments, the pore is a protein ion channel. Protein ion channels are naturally occurring proteins or derivatives thereof having a biological function. In some embodiments, the ion channel is produced by bacteria. Suitable protein ion channels include, but are not limited to, *Staphylococcus aureus* alpha-hemolysin, *Bacillus anthracis* protective antigen 63, gramicidin, MspA *(Mycobacterium smegmatis),* OmpF porin, Kapton, OmpG, and ClyA *(Salmonella typhimurium),* a non-naturally occurring compound, and derivatives thereof. The ion channel may also be a synthetic, or non-naturally occurring compound. Suitable ion channels are disclosed in U.S. Patent No. 7,504,505.

As described herein, an artificial membrane of the disclosure comprises a lipid bilayer that is linked to an external biomolecule. An "external" biomolecule is one that is not part of the artificial membrane *per se,* but is later associated with the artificial membrane through a linkage. Such a linkage, in various embodiments, is effected through a protein bridge (see, *e.g.,* Figure 1). In some embodiments, the linkage comprises a non-protein bridge (*e.g.,* direct covalent bonding of filament side chains to lipid head groups). Further embodiments comprise a non-protein polymer tether that allows controllable spacing between the membrane and the filamentous network. See, for example, U.S. Patent No. 7,504,505. In some embodiments, the number of linkages to the membrane is varied. The number of linkages to the membrane may be expressed as a number per unit area. In some embodiments, the protein bridge comprises (i) biotin and streptavidin; (ii) spectrin; (iii) avidin, neutravidin, or a biotin binding protein; (iv) a bridge protein from the ERM family (e.g., ezrin, radixin, or moesin); (v) a bridge protein from the formin family (e.g., myosin I, integrin, tensin, catenin (alpha-, beta-, or gamma-); (vi) a transmembrane glycoprotein (e.g., CD44); or (iv) digoxygenin and an antibody directed against digoxygenin. In general, the protein bridge may comprise any polypeptide and an antibody directed against the polypeptide. The disclosure further contemplates the use of any of several filament stabilizer molecules to further enhance stability and mechanical properties of the filamentous network. Stabilizer molecules bind along the side of a filament and may or may not participate in filament-filament linkages. Examples of actin-stabilizing molecules are tropomyosin and phalloidin. An example of a microtubule-stabilizing molecule is taxol. Further examples of microtubule stabilizer proteins are microtubule-associated proteins (MAPs), and include but are not limited to tau and MAP-2. In further embodiments, the disclosure contemplates the use of filament polymerization enhancers. Polymerization enhancers bind to monomer units to initiate and accelerate filament growth while also enhancing the mechanical properties of the external filamentous network. Examples of polymerization enhancers include but are not limited to XMAP215 (relevant to microtubules), gamma-TuRC (relevant to microtubules), formin (relevant to actin), and profilin (relevant to actin).

In further embodiments, the linkage comprises a bridge-forming molecule that enable networking (crosslinking) inside the external filamentous network. Such molecules enable filament-filament linkages, as opposed to filament-membrane linkages. By way of example, molecules such as streptavidin, avidin, or neutravidin can serve two purposes: (i) as a bridge to link the external filamentous network to a membrane (*e.g.,* a biotinylated membrane); and (ii) as a potential actin-actin crosslinker (*i.e.,* a linker that is inside the external filamentous network). Thus, creating multiple actin layers by bridging actin filaments one molecular layer at a time (or using filament-to-filament bridges to increase the 2D density of a sub-molecular layer) is contemplated herein. In further embodiments, a protein from the gelsolin family (*e.g.,* villin) is contemplated for use in forming bridges within the filamentous network. Additionally, fascin, fimbrin, alpha-actinin, spectrin, filamin, dystrophin, ARP complex, gamma-TuRC, and filaggrin are actin crosslinkers or binding proteins contemplated for use according to the disclosure. Plectin acts as a linker between all three major categories of cytoskeletal filaments (*i.e.,* actin filaments, microtubules, and intermediate filaments). In still further embodiments, links within the filamentous network comprise modified nucleotides, oligosaccharides, proteins, polyelectrolytes, or peptide strands. Any type of chemical linkage used to join individual filaments (e.g., via covalent, non-covalent, or ionic bonds) for molecular level control of the 2D and 3D filament density and branching structure within the filamentous network is contemplated by the disclosure.

Molecules useful in the devices and methods of the disclosure include, but are not limited to, a polypeptide, a peptide, an oligonucleotide, an oligosaccharide, a polymer, or a combination thereof. A "polymer" as used herein when referring to a molecule useful in a device of the disclosure is distinguished from, *e.g.,* a nucleic acid or protein, and a gel, hydrogel, and polyelectrolytes of both natural and synthetic origin. See: Noro et al., Soft Matter 8: 6416-6429 (2012) ; Laftah et al., Polymer Hydrogels: A Review, Polymer-Plastics Technology and Engineering 50: 1475-1486 (2011); and Costa et al., Chem. Soc. Rev. 43: 3453-79 (2014). As provided herein, the filamentous network is chemically linked to the bilayer (*e.g.,* lipid bilayer) of the device. In further embodiments, the chemical link is a covalent link. In some embodiments, the link is a non-covalent link.

Oligonucleotides contemplated by the present disclosure include DNA, RNA, modified forms and combinations thereof. The oligonucleotide, in various embodiments, is single stranded or double stranded. Accordingly, in some aspects, a device of the disclosure is linked to an external oligonucleotide that comprises DNA. In some embodiments, the DNA is double stranded, and in further embodiments the DNA is single stranded. In further aspects, a device of the disclosure is linked to an external oligonucleotide that comprises RNA, and in still further aspects a device of the disclosure is linked to an external oligonucleotide that comprises double stranded RNA. The term "RNA" includes duplexes of two separate strands, as well as single stranded structures. Single stranded RNA also includes RNA with secondary structure.

An "oligonucleotide" is understood in the art to comprise individually polymerized nucleotide subunits. The term "nucleotide" or its plural as used herein is interchangeable with modified forms as are known in the art. In certain instances, the art uses the term "nucleobase" which embraces naturally-occurring nucleotide, and non-naturally-occurring nucleotides which include modified nucleotides. Thus, nucleotide or nucleobase means the naturally occurring nucleobases adenine (A), guanine (G), cytosine (C), thymine (T) and uracil (U). Non-naturally occurring nucleobases include, for example and without limitations, xanthine, diaminopurine, 8-oxo-N6-methyladenine, 7-deazaxanthine, 7-deazaguanine, N4,N4-ethanocytosin, N',N'-ethano-2,6-diaminopurine, 5-methylcytosine (mC), 5-(C3-C6)-alkynyl-cytosine, 5-fluorouracil, 5-bromouracil, pseudoisocytosine, 2-hydroxy-5-methyl-4-tr- iazolopyridin, isocytosine, isoguanine, inosine and the "non-naturally occurring" nucleobases described in Benner et al., U.S. Pat. No. 5,432,272 and Susan M. Freier and Karl-Heinz Altmann, 1997, Nucleic Acids Research, vol. 25: pp 4429-4443. The term "nucleobase" also includes not only the known purine and pyrimidine heterocycles, but also heterocyclic analogues and tautomers thereof. Further naturally and non-naturally occurring nucleobases include those disclosed in U.S. Pat. No. 3,687,808 (Merigan, et al.), in Chapter 15 by Sanghvi, in Antisense Research and Application, Ed. S. T. Crooke and B. Lebleu, CRC Press, 1993, in Englisch et al., 1991, Angewandte Chemie, International Edition, 30: 613-722 (see especially pages 622 and 623, and in the Concise Encyclopedia of Polymer Science and Engineering, J. I. Kroschwitz Ed., John Wiley & Sons, 1990, pages 858-859, Cook, Anti-Cancer Drug Design 1991, 6, 585-607). In various aspects, oligonucleotides also include one or more "nucleosidic bases" or "base units" which are a category of non-naturally-occurring nucleotides that include compounds such as heterocyclic compounds that can serve like nucleobases, including certain "universal bases" that are not nucleosidic bases in the most classical sense but serve as nucleosidic bases. Universal bases include 3-nitropyrrole, optionally substituted indoles (*e.g.*, 5-nitroindole), and optionally substituted hypoxanthine. Other desirable universal bases include, pyrrole, diazole or triazole derivatives, including those universal bases known in the art.

Modified nucleotides are described in EP 1 072 679 and WO 97/12896. Modified nucleotides include without limitation, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Further modified bases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzox- azin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified bases may also include those in which the purine or pyrimidine base is replaced with other heterocycles, for example 7-deaza-adenine, 7-deazaguanosine, 2-aminopyridine and 2-pyridone. Additional nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, those disclosed by Englisch et al., 1991, Angewandte Chemie, International Edition, 30: 613, and those disclosed by Sanghvi, Y. S., Chapter 15, Antisense Research and Applications, pages 289-302, Crooke, S. T. and Lebleu, B., ed., CRC Press, 1993. Certain of these bases are useful for increasing the binding affinity and include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2° C and are, in certain aspects combined with 2'-O-methoxyethyl sugar modifications. See, U.S. Pat. Nos. 3,687,808, U.S. Pat. Nos. 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,750,692 and 5,681,941.

Methods of making oligonucleotides of a predetermined sequence are well-known. See, *e.g.*, Sambrook et al., Molecular Cloning: A Laboratory Manual (2nd ed. 1989) and F. Eckstein (ed.) Oligonucleotides and Analogues, 1st Ed. (Oxford University Press, New York, 1991). Solid-phase synthesis methods are preferred for both polyribonucleotides and polydeoxyribonucleotides (the well-known methods of synthesizing DNA are also useful for synthesizing RNA). Polyribonucleotides can also be prepared enzymatically. Non-naturally occurring nucleobases can be incorporated into the oligonucleotide, as well. See, *e.g.*, U.S. Patent No. 7,223,833; Katz, J. Am. Chem. Soc., 74:2238 (1951); Yamane, et al., J. Am. Chem. Soc., 83:2599 (1961); Kosturko, et al., Biochemistry, 13:3949 (1974); Thomas, J. Am. Chem. Soc., 76:6032 (1954); Zhang, et al., J. Am. Chem. Soc., 127:74-75 (2005); and Zimmermann, et al., J. Am. Chem. Soc., 124:13684-13685 (2002).

Oligonucleotides contemplated for use in the devices and methods of the disclosure are from about 5 nucleotides to about 10,000 nucleotides in length. More specifically, in some embodiments a device of the disclosure is linked to an external oligonucleotide that is about 5 to about 9000, about 5 to about 8000, about 5 to about 7000, about 5 to about 6000, about 5 to about 5000, about 5 to about 4000, about 5 to about 3000, about 5 to about 2000, about 5 to about 1000, about 5 to about 900, about 5 to about 800, about 5 to about 700, about 5 to about 600, about 5 to about 500, about 5 to about 400, about 5 to about 300, about 5 to about 200, about 5 to about 100, about 5 to about 90 nucleotides in length, about 5 to about 80 nucleotides in length, about 5 to about 70 nucleotides in length, about 5 to about 60 nucleotides in length, about 5 to about 50 nucleotides in length about 5 to about 45 nucleotides in length, about 5 to about 40 nucleotides in length, about 5 to about 35 nucleotides in length, about 5 to about 30 nucleotides in length, about 5 to about 25 nucleotides in length, about 5 to about 20 nucleotides in length, about 5 to about 15 nucleotides in length, about 5 to about 10 nucleotides in length, and all oligonucleotides intermediate in length of the sizes specifically disclosed to the extent that the oligonucleotide is able to achieve the desired result. Accordingly, oligonucleotides of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1,000, 2,000, 3,000, 4,000, 5,000, 6,000, 7,000, 8,000, 9,000, 10,000, or more nucleotides in length are contemplated. In further embodiments, oligonucleotides of at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1,000, at least 1,500, at least 2,000, at least 2,500, at least 3,000, at least 3,500, at least 4,000, at least 4,500, at least 5,000, at least 5,500, at least 6,000, at least 6,500, at least 7,000, at least 7,500, at least 8,000, at least 8,500, at least 9,000, at least 9,500, or at least 10,000 nucleotides in length are contemplated.

As used herein a "polypeptide" refers to a polymer comprised of amino acid residues. In some aspects of the disclosure, a device is linked to an external polypeptide as described herein. Polypeptides are understood in the art and include without limitation an antibody, an enzyme, a structural polypeptide and a hormone.

Polypeptides of the present disclosure may be either naturally occurring or non-naturally occurring. Naturally occurring polypeptides include without limitation biologically active polypeptides (including antibodies) that exist in nature or can be produced in a form that is found in nature by, for example, chemical synthesis or recombinant expression techniques. Naturally occurring polypeptides also include lipoproteins and post-translationally modified proteins, such as, for example and without limitation, glycosylated proteins. Non-naturally occurring polypeptides contemplated by the present disclosure include but are not limited to synthetic polypeptides, as well as fragments, analogs and variants of naturally occurring or non-naturally occurring polypeptides as defined herein. Non-naturally occurring polypeptides also include proteins or protein substances that have D-amino acids, modified, derivatized, or non-naturally occurring amino acids in the D- or L- configuration and/or peptidomimetic units as part of their structure. The term "protein" typically refers to large polypeptides. The term "peptide" generally refers to short (*e.g.*, about 50 amino acids or less) polypeptides.

Non-naturally occurring polypeptides are prepared, for example, using an automated polypeptide synthesizer or, alternatively, using recombinant expression techniques using a modified oligonucleotide which encodes the desired polypeptide.

In some embodiments, the polypeptide is a cytoskeletal protein. See, e.g., Alberts, Johnson, Lewis, Morgan, Raff, Roberts, Walter, Wilson, Hunt, Molecular Biology of the Cell, Ch. 16, 6th ed., Garland, 2015. In further embodiments, the cytoskeletal protein is an actin filament, catenin, an intermediate filament protein, a microfilament protein, or a microtubule protein. In some embodiments, the catenin is alpha catenin, beta catenin, or gamma catenin. In further embodiments, the intermediate filament protein is desmin, glial fibrillary acidic protein, keratin, nestin, or vimentin. In some embodiments, the microfilament protein is actin, actinin, filamin, gelsolin, myosin, profilin, tensin, tropomyosin, troponin, or a derivative thereof. In some embodiments, the microtubule protein is dynein, tubulin, or kinesin. In some embodiments of the disclosure, a multiple-layer device is contemplated. Controlled multiple-layering is contemplated for all filamentous networks comprising, *e.g.*, proteins, oligonucleotides, polymer gels, hydrogels, and polyelectrolytes. Use of cytoskeletal proteins in devices described herein is important for constructing multiple networked layers. Multiple layers allow for the extension of the 2D-form into a 3D-form for the device. In some embodiments, a 3D network provides further increased resistance to mechanical stress as well as protection from membrane dehydration. The properties of 3D networkability translate into commercially significant features such as extended shelf-life of the device, mechanical durability, field-worthiness, transportability, and reusability.

As used herein a "fragment" of a polypeptide is meant to refer to any portion of a polypeptide or protein smaller than the full-length polypeptide or protein expression product.

As used herein an "analog" refers to any of two or more polypeptides substantially similar in structure and having the same biological activity, but can have varying degrees of activity, to either the entire molecule, or to a fragment thereof. Analogs differ in the composition of their amino acid sequences based on one or more mutations involving substitution, deletion, insertion and/or addition of one or more amino acids for other amino acids. Substitutions can be conservative or non-conservative based on the physico-chemical or functional relatedness of the amino acid that is being replaced and the amino acid replacing it.

As used herein a "variant" refers to a polypeptide, protein or analog thereof that is modified to comprise additional chemical moieties not normally a part of the molecule. Such moieties may modulate, for example and without limitation, the molecule's solubility, absorption, and/or biological half-life. Moieties capable of mediating such effects are disclosed in Remington's Pharmaceutical Sciences (1980). Procedures for coupling such moieties to a molecule are well known in the art. In various aspects, polypeptides are modified by biotinylation, glycosylation, pegylation, and/or polysialylation.

Fusion proteins, including fusion proteins wherein one fusion component is a fragment or a mimetic, are also contemplated. A "mimetic" as used herein means a peptide or protein having a biological activity that is comparable to the protein of which it is a mimetic.

Also contemplated by the disclosure are devices that are linked to an external phospholipid.

Oligosaccharides useful in the devices and methods disclosed herein include any carbohydrates comprising between about two to about ten monosaccharides or more connected by either an alpha- or beta-glycosidic link. Oligosaccharides are found throughout nature in both the free and bound form.

## Methods of using a device of the disclosure

Various methods for using the devices of the disclosure are contemplated. In some aspects, methods of analyzing a target polymer are provided comprising contacting the target polymer to a device of the disclosure, allowing the target polymer to move with respect to the at least one pore present in the device to produce a signal, and monitoring the signal corresponding to the movement of the target polymer with respect to the pore, thereby analyzing the target polymer. In some embodiments, the signal monitoring comprises measuring a monomer-dependent characteristic of the target polymer while the target polymer moves with respect to the pore. In further embodiments, the monomer dependent property is the identity of a monomer or the number of monomers in the polymer. The target polymer, in various embodiments, is an oligonucleotide, a peptide, or a polypeptide, each as described herein. In some embodiments, the analyzing comprises a chemical characterization. In further embodiments, the chemical characterization is a characterization of DNA, a synthetic polymer, a small molecule, or an ion. In some embodiments, the characterization of DNA comprises nucleotide sequencing or genotyping.

Thus, in some embodiments the entire structure including the membrane, aperture, and an ion channel are useful for analysis of polymers. In some embodiments, the analysis comprises DNA and other polynucleotide sequencing. An electrolyte solution containing the DNA is placed on one side of the membrane. Electrolyte is also placed on the other side of the membrane. A voltage is applied through the electrolytes and across the membrane. This causes a DNA strand to gradually pass through the membrane. As the strand passes through, the current passing through the membrane is measured. The current is affected by the number and identity of the nucleotides presently in the pore. When using protein ion channels, there is typically more than one nucleotide in the pore. The identity of each nucleotide is determined from several current measurements as the nucleotide passes through the pore. A synthetic pore may be short enough to hold only one nucleotide. This simplifies the sequencing, as each nucleotide identification is determined from a single current measurement.

In some embodiments, the device further comprises a molecular motor. The molecular motor, in some embodiments, is adjacent to a pore of the device, and the molecular motor is capable of moving a target polymer with respect to the pore. By way of example, a target polymer can be passed through a molecular motor tethered to the surface of a device or embedded in a device, thereby bringing units of the target polymer sequentially to a specific location, preferably in interactive proximity to an agent. Agents contemplated herein include but are not limited to electromagnetic radiation, a quenching source and a fluorescence excitation source. Individual units of the target polymer interact with the agent to produce a detectable signal, and the signals resulting from said interaction are sequentially detected to analyze the polymer. According to some embodiments of the disclosure, individual units of the target polymer are labeled with a fluorophore.

A molecular motor is a compound such as polymerase, helicase, or actin which interacts with the polymer and is transported along the length of the polymer past each unit. In further embodiments, the molecular motor comprises a DNA polymerase, a RNA polymerase, a ribosome, or an exonuclease. In still further embodiments, the DNA polymerase is selected from *E. coli* DNA polymerase I, *E. coli* DNA polymerase I Large Fragment (Klenow fragment), phage T7 DNA polymerase, Phi-29 DNA polymerase, *Thermus aquaticus* (Taq) DNA polymerase, *Thermus flavus* (Tfl) DNA polymerase, *Thermus Thermophilus* (Tth) DNA polymerase, *Thermococcus litoralis* (Tli) DNA polymerase, *Pyrococcus furiosus* (Pfu) DNA polymerase, *Bacillus stearothermophilus* (Bst) DNA polymerase, AMV reverse transcriptase, MMLV reverse transcriptase, and HIV-1 reverse transcriptase.

In some embodiments, the RNA polymerase is selected from T7 RNA polymerase, T3 RNA polymerase, SP6 RNA polymerase, and *E. coli* RNA polymerase. In some embodiments, the exonuclease is selected from exonuclease Lambda, T7 Exonuclease, Exo III, RecJ, Exonuclease, Exo I, and Exo T. In some embodiments, the helicase is selected from *E. coli* bacteriophage T7 gp4 and T4 gp41 gene proteins, *E. coli* protein DnaB, *E. coli* protein RuvB, and *E. coli*

protein rho.

In some embodiments, methods of analyzing a target polymer further comprise altering the rate of movement of the polymer before, during, or after the signal monitoring.

In general, lipid bilayers create distinct spatial compartments where it is critical to isolate molecular ingredients, or physical conditions, on each side of the partition. Thus, in addition to nanopore sensing, and in various aspects and embodiments, the disclosure contemplates additional uses of the devices disclosed herein. For example and without limitation, any of the devices described herein are contemplated for use in ion channel electrophysiology, liposome drug delivery, and the engineering of synthetic cells. In ion channel electrophysiology, different chemicals and electrical potentials are sequestered. In liposome drug delivery, pharmaceutical products are confined within the protective interior of a liposome. In synthetic cells, complex metabolic pathways, organelles, and other structures are kept isolated from the surrounding environment. For proper function in each of the uses described herein, the strength, durability, and integrity of the lipid bilayer is of central concern. Each of these features can be enhanced by use of the technology disclosed herein. Indeed, the devices disclosed herein (including but not limited to devices comprising multiple layers) are contemplated for use in any application that utilizes a lipid bilayer partition.

To the extent that any meaning or definition of a term in this document conflicts with any meaning or definition of the same term in a document cited herein, the meaning or definition assigned to that term in this document shall govern.

## EXAMPLES

### Example 1

The following example demonstrates the mechanical enhancements and electrical properties of membranes when a phalloidin-stabilized f-actin support structure is chemically linked to the lipid bilayer. After formation of the scaffolding, changes were assessed by applying trans-membrane pressure and measuring the membrane area expansion and elastic modulus. The structure was also characterized electrically and with optical microscopy. Lastly, the support network was demonstrated to permit direct diffusional access to the bilayer and maintain the normal electrical conductance of the alpha-hemolysin nanopores inserted into the supported membrane.

### Materials and Methods

**Lipids.** Diphytanoyl 1,2,-diacyl-sn-glycero-3-[phospho-L-serine] (DiPhyPC) and N-Biotinyl-PE 16:0 were purchased from Avanti Polar Lipids (Alabaster, AL; Product No. 870285). All lipid materials were dissolved in either hexadecane (Aldrich, Milwaukee, WI) or pentane (VWR, Bridgeport, NJ). A 5:1 mole ratio of DiPhyPC:N-Biotinyl-PE was used to create lipid membranes.

**Filamentous Actin.** Rabbit skeletal muscle actin monomers [Cytoskeleton Inc., >99% purity] were polymerized into filamentous form (f-actin) from monomers using varying ratios of globular actin (G-actin) [AR05], biotinylated actin [AB07], and rhodamine-phalloidin [PHDR1]. Phalloidin is a seven amino acid peptide toxin from the mushroom *Amanita phalloides* that binds to the helical structure of f-actin and was dissolved in methanol from lyophilized powder. Actin monomers (5:1 mole ratio, G-actin:biotin actin) were reconstituted in a buffer of 0.2 mM $CaCl_2$ and 5 mM Tris-HCl, pH 8.0 and placed on ice for one hour. Polymerization was then initiated by the addition of KCl, $MgCl_2$, and Adenosine Triphosphate (ATP). In addition, a 10x molar excess of phalloidin was also added to the mixture to promote filament elongation and reduce the critical concentration required for actin polymerization. The final f-actin growth media contained 2 $\mu$M actin (5:1 G-actin/biotin actin), 20 $\mu$M phalloidin, 1 M KCl, 10 mM Tris-HCl (pH 7.5-8.0), 2 mM $MgCl_2$, 1 mM ATP, 0.1 mM $CaCl_2$ and <2% methanol v/v. The reaction mixture was left on ice overnight before use.

**Polymerization Assay.** Before utilizing f-actin to stabilize membranes, growth conditions were tested via fluorescence spectroscopy to insure proper actin polymerization and also to determine the longevity of f-actin stability. For these studies, pyrene-labeled actin, biotinylated actin, and G-actin were mixed using the growth media and conditions described above. Reorientation of the pyrene-labeled monomers upon polymerization resulted in an increase of pyrene fluorescence intensity. Bulk polymerization in a 1 mL solution was followed in a cuvette using a fluorescence spectrometer (355 nm ex, 400-410 nm em). The kinetics of the polymerization and results from the longevity study are detailed herein.

**Streptavidin Linker.** Streptavidin [Sigma-Aldrich, S4762] was reconstituted in 100 mM Tris-HCl, pH 7.5 to 1 mg/mL. Before application to biotinylated lipid membranes, an aliquot of streptavidin was mixed with an equal volume of 2 M KCl. This increased the solution density to match (or be slightly higher than) the solution surrounding the lipid membrane. This quickly sinks the streptavidin containing solution so that it contacts the membrane at high concentration; it also eliminates the need for stirring after delivery to ensure adequate membrane coverage. Figure 1A depicts the molecular structure connecting the lipid membrane to an interwoven f-actin network. Amine-containing residues within the actin monomer are derivatized with biotin. Biotin is also chemically joined to lipid headgroups through a short flexible hydrocarbon chain. Bridging the two forms of biotin with a streptavidin tetramer effectively links the f-actin network to the stable hydrophobic interior of the membrane.

αHL Ion Channels. αHL was amplified by PCR from a DNA template (pBR322 containing a 3 kb EcoRI/HindIII insert including αHL) and subcloned into pET-2T-parallel [Chandler et al., Langmuir: the ACS journal of surfaces and colloids 2004, 20, 898-905] using the NcoI and SpeI sites. The entire sequence of αHL was confirmed using DNA sequencing. Expression of the (His6)-fusion protein was in BL21(DE3) cells (Novagen, Madison, WI). Briefly, cells at an OD600 of 0.6-0.7 were induced with 1 mM IPTG after which they were grown for 3 hours at 37 °C. Pelleted cells were lysed in BPER buffer (Pierce, Rockford, IL) followed by centrifugation at 14,000 rpm for 30 minutes. The soluble His-tagged αHL was purified using HiTrap resin (Amersham Biosciences, Piscatawy, NJ). After washing the resin with buffer (20 mM Tris pH 8.0, 100 mM KCI), fusion protein was eluted with buffer containing imidazole. Removal of the His6 tag was accomplished by rTEV protease (Invitrogen, Carlsbad, CA) during dialysis in 20 mM Tris pH 8.0, 100 mM KCI, 1 mM DTT, 0.5 mM EDTA. SDS-PAGE verified the molecular weight of αHL and revealed that the αHL accounted for at least 95% of the protein present in the sample. The yield of αHL was approximately1 mg from 50 ml of cell culture.

Optical Imaging. Creation of f-actin filaments was verified by imaging via confocal fluorescence microscopy on glass slides using 514-nm laser excitation and 565-580 nm emission band. Figure 1B illustrates membrane chamber used in both confocal and widefield imaging experiments. The optical train consists of a confocal microscope that was modified from previous work [Chandler et al., Langmuir: the ACS journal of surfaces and colloids 2004, 20, 898-905] to enable simultaneous electrical and optical recordings. The microscope was configured in an upright geometry and used water-immersion optics to interrogate membranes formed on a horizontal bilayer sample chamber, and also to estimate the length of the f-actin filaments spread on a microscope slide. Confocal images (10 × 10 $\mu$m) were acquired using mechanical scanning and 100-nm step resolution. Widefield images were acquired using an intensified CCD camera (ICCD) with 0.5-1 $\mu$m resolution in both the bright-field and fluorescence modes.

Microscope Slide Preparation. Slides were prepared by cleaning with water and incubating for 30 minutes with a small droplet of streptavidin at 1 mg/mL. After a wash with buffer, the f-actin growth solution was applied and allowed to bind to the streptavidin-coated surface. Following a gentle rinse with buffer, slides were imaged with a confocal microscope.

Lipid Membrane Formation. Unsupported lipid bilayers were formed on a small Teflon aperture with a modified version of the Mueller-Rudin technique [Mueller et al., Nature 1962, 194, 979-980]. A horizontal bilayer chamber, similar to that described elsewhere [Burden et al., J Phys Chem B 2000, 104, 6103-6107], was placed under an upright microscope for imaging with a low magnification microscope objective (Figure 1B). A constriction was formed between an upper and lower solution-filled chamber (1 M KCI, 5 mM HEPES, pH 7.5) by forcing a steel needle tip through a Teflon plug until the sharpened end punctured the surface. After needle extraction, the remaining conduit formed a taper that varied from 20-100 $\mu$m in diameter with an approximately circular opening at the plug's surface. Aperture diameters typically varied from 30-50 $\mu$m. Bilayers were prepared by first allowing a lipid in pentane solution to dry on the aperture surface. The upper and lower chambers were then filled with aqueous buffer solution containing 1 M KCI and bilayers were created by stroking lipid in hexadecane across the aperture using a small bubble formed at the end of a pipette tip.

f-actin was chemically linked to the bilayer using streptavidin. Following bilayer formation, a small aliquot of streptavidin was applied over the membrane surface via pipette. After approximately a 5 min incubation period for streptavidin to bind with biotinylated lipids, the chamber was rinsed (approximately 3-5 volume exchanges) with KCI buffer. f-actin polymer filaments containing 20% mole ratio biotinylated monomers were then applied using a truncated pipette tip and incubated for 5 minutes before executing another chamber rinse. The same rinsing procedure was used to create monolayers of f-actin on streptavidin-coated glass microscope slides for filament length measurement.

Apparatus. A custom apparatus was constructed to perform combined electrical and optical recordings on horizontally oriented lipid bilayers. The system also enabled transmembrane pressure control and measurement. Figure 5 presents various components of the system. Figure 5A shows the transparent plexiglass chamber fitted with a white teflon plug, which separates two liquid-filled compartments. The top compartment (approximately 3 mL) remained open to the atmosphere to allow optical imaging. The bottom compartment (approximately 1 mL) resides inside the plexiglass chamber. A small, conically shaped conduit was fashioned through the plug by forcing a needle tip through the top surface of the Teflon to create an aperture that is 20-100 $\mu$m in diameter. Horizontal lipid membranes were suspended across this aperture. A differential pressure transducer acquires pressure readings from the bottom chamber through a hydraulic connection to the lower liquid-filled chamber. The lower compartment is also connected to tubing that runs out from the chamber to a 90 degree vertical elbow, which redirected the tubing upward, above the surface of the liquid in the top bilayer compartment. A portion of this segment was made out of large-diameter Tygon tubing. This portion formed a compression cavity, which was mounted against a rigid vertical support. A computer-controlled actuator compressed or expanded the compression cavity against the rigid support, thereby raising and lowering the height of solution in the vertical portion of the tubing. The pressure control mechanism is pictured from two angles in Figures 5A and 5B. All components were mounted on a custom-designed microscope translation stage that allowed positioning of the apparatus using both manual and piezo electric control. Figure 5C shows a low-magnification image of the top liquid-filled compartment and the surface of the Teflon plug illuminated from below. Image analysis software enabled characterization of the size and shape of apertures formed by the needle tip. Figure 5C shows a semicircular aperture with an intercept of 44 $\mu$m, an area of 1051 $\mu$m$^2$, and a Heywood circularity factor of 1.12. Figure 5D shows combined bright-field and fluorescence images of an actin-coated membrane suspended across this aperture. The method of producing and applying the filamentous actin mesh that provides mechanical support to the bilayer is described herein. Dim fluorescence (inset of Figure 5D arises from a large portion of the bilayer surface, with bright fluorescence generated by high-density regions of actin filaments spanning portions of the membrane, as well as the top surface of the Teflon plug.

**Bilayer Response to Applied Pressure**. The rupture of an uncoated lipid bilayer in response to a large single-step pressure increment was also demonstrated. These experiments demonstrated events that terminated data collection in typical capacitance vs. time measurements (*e.g.*, Figure 2A). Because the membrane is transparent, membrane rupture was visualized by depositing small polystyrene spheres (approximately 1 $\mu$m) in a loose layer over the aperture after bilayer formation. The polystyrene spheres blur the optical contrast, especially the distinction of the aperture rim, and produced a shadowed haze over the surface of the Teflon plug. However, the aperture rim can still be faintly located in the approximate middle of the illuminated field of view. The experiment began with a large pressure increment (>200 Pa) applied to the membrane and the corresponding upward expansion of the bilayer. The expansion process was optically invisible, but was readily detected via capacitance monitoring. At approximately 14 seconds, the bilayer reaches a breaking point and membrane rupture occurred with a sudden expulsion of fluid from the tapered conduit below. The burst of clear solution disrupted the uniform layering of the beads, producing a visible shock wave in the vicinity of the aperture.

**Elasticity Modulus: Nonlinearity, Comparison to Previous Experiments, Analysis Assumptions**. Figure 6 demonstrates the non-linearity of the in-plane elasticity modulus ($E_\parallel$) as the area expansion grows large in comparison to measurements from other investigators [Hianik et al., In Bilayer Lipid Membranes: Structure and Mechanical Properties; Kluwer Academic Publishers: Norwell, MA, 1995, pp 965.)]. $E_\parallel$ was computed as the slope of measured pressure vs. h($\Delta$C/C)/R, following theory developed and described herein. As shown in Figure 6A, $E_\parallel$ possesses its largest value when the pressure is low and the corresponding area expansion is small.

Both the trend and absolute magnitude of $E_\parallel$ computed in our experiments for large area expansions (*i.e.*, >1%) compare favorably with other reports. These reports employ similar lipid/solvent systems without additional coatings, but at area expansions of <1%. Figure 6B shows $E_\parallel$ values (closed squares) as a function of membrane surface expansion in comparison to data taken from a report by Hianik for smaller area expansions (open squares) [Hianik et al., In Bilayer Lipid Membranes: Structure and Mechanical Properties; Kluwer Academic Publishers: Norwell, MA, 1995, pp 965]. Hianik's experiments were performed using a pressure oscillation technique that involved a small change in membrane area. For small membrane expansion, changes in the out-of-plane radius of curvature (R) were assumed to be insignificant [Wobschall et al., Journal of Colloid and Interface Science 1971, 36, 385-396; Passechnik et al., Biofizika 1973, 18, 655]. It was noted that the determinations of $E_\parallel$ (using a similar but modified theoretical analysis that necessitates estimates for R fit a continuous trend with respect to Hianik's results as the %$\Delta$ Area transitions above 1%. This continuity bolsters confidence that the analysis produced meaningful absolute values of $E_\parallel$, despite the fact that the apertures were not perfectly circular. Lastly, for relative comparisons of $E_\parallel$ between coated and uncoated membranes, it was assumed that the relatively small diameter of actin filaments (approximately 6 nm) and the open mesh structure of the f-actin web (*i.e.*, incomplete monolayer coverage), does not significantly thicken the membrane. Thus, the estimate of h (*i.e.*, the average membrane thickness) remains identical (5 nm) for all $E_\parallel$ values computed in this study.

**Filament Growth Kinetics and Longevity.** Figures 7A and 7B show f-actin polymerization kinetics and the resistance to degradation of bulk actin filaments. Polymerization assays were performed as described by the actin vendor (Cytoskelton.com) and as briefly summarized herein. The data shown in Figure 7 result from the polymerization of pyrene-labeled actin, biotinylated actin, G-actin, and phalloidin after mixing with growth media. In this assay, streptavidin was not utilized. Reorientation of the pyrene-labeled monomers upon polymerization increased pyrene fluorescence intensity, allowing the status of filament elongation to be monitored. As can be seen in Figure 7A, polymerization occurred rapidly after initiation by the addition of KCl, MgCl$_2$, and adenosine triphosphate. Full polymerization (*i.e.*, fluorescence maximum) occurred within a few hundred seconds. Photobleaching causes the slight decrease in intensity over the ensuing approximately 1500s.

Figure 7B shows fluorescence intensity from a bulk polymerized sample that was monitored over the course of many months. Phalloidin-stabilized filaments remained stable for long periods of time with little deterioration. Over the duration of the measurements, the decrease in fluorescence intensity represented approximately a 7% degradation of the initial polymerized network.

**Electrical Recordings.** Electrical potentials were applied across the aperture using a DC-10 MHz waveform generator (Hewlett Packard 33120A, Palo Alto, CA) and Ag/AgCl electrodes (In Vivo Metric, Ukiah, CA) which were placed above and below the Teflon plug. The ionic current was amplified by a factor of 10$^6$-10$^9$ using a custom-built current-to-voltage converter and low-pass filtered using an 8-pole Bessel active filter (Frequency Devices 9002, Haverhill, MA). Data were digitized at 10 Hz using a 12-bit analog-to-digital converter (PCI-MIO-16E-1, National Instruments, Austin, TX) and were analyzed with software written in LabVIEW. The bilayer chamber and the microscope were isolated from sound and light, as well as external electrical fields, by an enclosed Faraday cage.

*Capacitance Recordings.* A time course of capacitance readings was used to signal successful bilayer formation. Capacitance measurements also provide a means to monitor the bilayer response to transmembrane pressure, as the area of the membrane changes in response to stress. A triangle wave (400 Hz, 100 mV peak-peak) applied across the aperture opening generated a corresponding output square wave after membrane formation. The amplitude of this signal was converted into a calibrated capacitance value, which was continuously logged at 1 Hz. Typical capacitance readings for properly formed planar bilayers on our apertures ranged from 15-30 pF, depending upon the aperture size. Given the area of the aperture as determined by image analysis, a specific capacitance of approximately 0.7 $\mu$F/cm$^2$ was determined. Others have reported specific capacitance values from 0.4-1 $\mu$F/cm$^2$ for functional bilayer membranes [Niles et al., Biophys. J. 1988, 53, 327-335; White et al., Biochem. Biophys. Acta 1973, 323, 7-22].

*Current Recordings.* A 120 mV applied DC electrical potential enables measurement of open $\alpha$HL channels in the membrane. Protein monomers were added via pipette to the top solution chamber. After addition, several minutes was permitted to allow protein migration, channel assembly, and pore opening. Current measurements were acquired using a 500 Hz low-pass filter and were logged at 10 Hz.

**Pressure Control and Measurement.** One portal of a differential pressure transducer (Honeywell, 164PC01D37) was connected via buffer-filled Tygon tubing to the bottom chamber of the bilayer apparatus. Output voltage from the transducer is directly proportional to pressure from 0-10 inches H$_2$O (0-2490 Pa) at 20° C. The second portal of the pressure transducer was left open to ambient atmospheric conditions.

The bottom of the bilayer chamber was connected to a second segment of tubing filled with buffer. This tubing arm formed a 90 degree vertical elbow several centimeters (inches) away from the chamber and extended upward several more centimeters (inches), well above the liquid level contained in the top bilayer chamber. The bottom portion of this arm consisted of compressible 0.635 cm (¼-inch) i.d. Tygon tubing that contained a relatively large volume of buffer. A smaller diameter tubing (1 mm i.d.) extended above the elbow by several centimeters (inches), with the end of the tube left open to the atmosphere. The ratio of large and small diameter tubing volumes was designed so that fluid displacement in the large diameter tube would cause the fluid level in the narrow tube to rise upward, thereby increasing the pressure in the bottom chamber and the connected portal of the pressure transducer. By positioning the flexible large-diameter tube against a fixed barrier, the height of the water level above the top chamber in the small-diameter tube could be controlled via compression from a stepper-motor-controlled actuator. Raising the level of solution in the tube above the equilibrium level for the top chamber increased the transmembrane solution pressure, applying a controlled and reversible force to the lipid bilayer spanning the aperture.

Calibration of the pressure control and measurement system was performed using a sealed Teflon plug (*i.e.*, no aperture or bilayer) against the measured vertical displacement of water above the equilibrium level. The system demonstrated a linear response with negligible hysteresis, as long as all air bubbles were flushed from the system. Changes in pressure could be measured below $\Delta$2 Pa.

**Results and Discussion**

**Linked f-actin Surface Coverage.** After the formation of a bilayer, we add components for the support network in a stepwise manner. Each step is followed by a rinse of approximately 3-5 chamber volumes (9-15 mL). Presumably, the available biotin binding sites are nearly filled by the streptavidin addition. Polymerized f-actin is then added and given several minutes to bind before the second rinse commences. Confocal fluorescence microscopy reveals that this layering procedure typically results in less than a monolayer coverage (Figure 1C and 1D), as individual filaments can be readily distinguished stretching over the surface.

A typical mesh applied to glass is shown in Figure 1C. This shows a distribution of polymer filament lengths that range from 3-15 $\mu$m. Mesh spacing differs from experiment to experiment depending on the effectiveness of the manual application from the pipette; however, inter-filament openings of 5-50 $\mu$m$^2$ were regularly observed. Figure 1D shows a brightfield image of an aperture in Teflon with its associated geometric specifications. The inset to Figure 1D was collected from the rim of the aperture via a confocal scan after a bilayer was suspended and derivatized with both streptavidin and f-actin. The Teflon and hexadecane annulus ring appeared to fluoresce at a level that competes with the intensity of single f-actin filaments. Filaments were seen covering the area around the aperture rim and the solvent annulus ring. Filaments also extended inwardly across the lipid bilayer. To assess the thickness of the filamentous netting, a z stack of images was collected. Filaments disappeared in scans collected 0.5-1 $\mu$m away from the surface, which is the approximate axial resolution of the confocal system. A z-stack of images collected using 50-nm axial step increments contains a sharply focused layer of polymer filaments at the surface and grows increasingly defocused with distance, without the appearance of additional in-focus filaments. This suggested that the support structure forms a primarily two-dimensional network.

**Temporal Response to Applied Pressure.** Figure 2 displays the results from several experiments where trans-aperture pressure was applied. Membrane expansion was monitored over time via capacitance and video recording.

Typically, experiments were continued until the membrane began to expand uncontrollably, or it irreversibly ruptured. Rupture events are visualized via video microscopy as a small burst of fluid is released upon membrane breakage.

Two styles of experiments were performed: single-step pressure tests and multi-step pressure tests. Single-step tests employ relatively high, but constant, applied pressure. Multi-step tests apply pressure in small step-wise increments, which enables a more controlled membrane area expansion and calculation of the in-plane membrane elasticity.

Bilayers with an f-actin mesh connected to one or both sides of the membrane were also investigated. For one-sided measurements, f-actin was applied via pipette from the top bilayer chamber, followed by a chamber rinse to sweep away excess material. For two-sided experiments, breakage was induced via the application of high voltage after the first addition of streptavidin and f-actin. A second aliquot of streptavidin and f-actin was then delivered near the open aperture, followed by an approximate 3-5 minutes incubation to allow material to settle within the aperture opening. A lipid bilayer was then reformed by manipulating a bubble from a pipette tip across the aperture. Membrane capacitance measurements confirmed the bilayer was of appropriate thickness (approximately 5 nm) before continuing with the experiment. After reformation, another aliquot of streptavidin and f-actin was applied to the top surface, each step followed by a chamber rinse. The presence of the supporting mesh on one or both sides of the membrane did not appear to alter bilayer capacitance values significantly. This arose from the openness of the filament network and its thin, near-2D structure.

As can be seen in Figure 2A, uncoated membranes expand to breakage rapidly upon the addition of 200 Pa of pressure. Membranes with one treated side respond with a much slower expansion, but still typically expand to breakage within seconds to minutes. However, membranes treated on both sides of the aperture yielded a flat capacitance response over time, indicative of the improved mechanical resilience conveyed by the supporting mesh. The observations suggested that it is possible for all three membrane types to remain intact for several hours in the absence of applied stress. However, under applied pressure, only membranes coated on both sides remained stable for multiple hours before the recordings were intentionally terminated.

Figures 2B-2D show multi-step pressure tests for an uncoated membrane and a membrane coated on one side only. A computer-controlled actuator (gray solid line) regulates the trans-aperture pressure increase in small steps, which is registered by the pressure transducer (black solid line). The actuator position, overlaid as a unitless quantity, shows a clear correlation between pressure application and the detected pressure increase. Commonly, a slight pressure loss follows a step increment, presumably due to membrane bulging or movement that permits a volume increase in the bottom chamber. Capacitance recordings begin with a value that is characteristic of a bilayer spanning the entire area of the aperture. This value increases in response to applied pressure, as the membrane bows upward and the area of the membrane expands. The storage of lipid molecules in the torus of a BLM assists expansion because additional material can be physically drawn into the bilayer as the area increases. Figures 2B and 2C show the change in capacitance (open circles) measured after the capacitance of the original (presumably flat) membrane has been established. Frequently, membranes are observed expanding to an equilibrium position in stepwise synchrony with applied pressure, particularly at the beginning of an experiment, when the area expansion is small. All multi-step experiments are continued until the rate of membrane expansion transitions into a region of continuous growth. In Figures 2B-2D, these "run-away" thresholds appear at approximately 125 s, approximately 255 s, and approximately 675 s, respectively (or at pressures of approximately 60, approximately 90 and approximately 150 Pa). The pressure at which this transition occurs varies from trial to trial by as much as approximately 30%, as an apparent random consequence of the size of the solvent torus, the size of the aperture, or the density of actin filaments spread over the membrane. However, application of the linked f-actin network to one, or both, sides of the bilayer consistently stiffened the membrane, allowing it to tolerate significantly higher pressures than its untreated counterpart.

**Theory of in-plane expansion modulus.** To compare the mechanical properties of bilayers under applied stress, a basic model of elastic bodies was adopted which assumes isotropic homogeneity of the membrane and allows calculation of the modulus of elasticity from readily observable parameters. Here, the theory summarized by Hianik [Hianik et al., In Bilayer Lipid Membranes: Structure and Mechanical Properties; Kluwer Academic Publishers: Norwell, MA, 1995; , pp 965] was modified for the experimental configuration used herein.

The area extension in a planar membrane presumes equal stress ($\sigma$) in the X and Y directions. The Young's elasticity modulus can be defined as

$$E_{\parallel} = \frac{\sigma_x + \sigma_y}{\Delta A / A_o} = \frac{2\sigma}{\Delta A / A_o} \qquad \textbf{Equation 1}$$

where $\sigma_x$ and $\sigma_y$ are applied stresses in the X and Y directions and $\Delta A / A_o$ is the change in the bilayer area ($\Delta A$) relative to the undistorted area ($A_o$). Here, $\Delta A / A_o = \Delta C / C_o$, since capacitance is a direct measure of bilayer area. The technique of measuring the modulus assumes membranes with spherical surfaces that bulge as a consequence of an applied hydrostatic pressure gradient ($p$). For a spherical membrane [Landau et al., In Theory of Elasticity; Nauka: Moscow, 1965]

$$p = h2\sigma/R \qquad \textbf{Equation 2}$$

thus,

$$p = {}^{hE_{\parallel}(\Delta A/A_o)}/{}_R = {}^{E_{\parallel}h(\Delta C/C_o)}/{}_R \qquad \textbf{Equation 3}$$

where $h$ represents the membrane thickness and $R$ is the radius of curvature that characterizes out-of-plane deformation.

In general, the surface area of a spherical segment is computed as the zone of a sphere [Weisstein, "Zone." From MathWorld--A Wolfram Web Resource. http://mathworld.wolfram.com/Zone.html (accessed October 9, 2017)]

$$A' = 2\pi R\left(\sqrt{R^2 - b^2} - \sqrt{R^2 - a^2}\right) \qquad \textbf{Equation 4}$$

where $a$ and $b$ are radii associated with the upper and lower boundaries of the zone and $R$ is the radius of the sphere. When adapted for the case of a circular membrane deformed from its initial planar geometry, $b \rightarrow 0$ and $a = r$, where $r$ is the radius of the supporting aperture. Thus, the out-of-plane radius of curvature of a bulging membrane can be computed as

$$R = {}^{(A'/2)}\big/{}_{\sqrt{A'\pi - r^2\pi^2}} \qquad \textbf{Equation 5}$$

Since the relative change in surface area is proportional to the capacitance change, $\Delta A/A_o = (A' - A_o)/A_o = \Delta C/C_o = (C' - C_o)/C_o$, $A'$ can be found by estimating $A_o$ from optical microscopy and scaling it by the measured relative capacitance change.

$$A' = A_o\Delta C/C_o \qquad \textbf{Equation 6}$$

thus,

$$R = {}^{\left((A_o\Delta C/C_o)/2\right)}\big/{}_{\sqrt{(A_o\Delta C/C_o)\pi - r^2\pi^2}} \qquad \textbf{Equation 7.}$$

To calculate $E_{\parallel}$ using Equation 3, the radius of the aperture ($r$) and initial area ($A_o$) were measured via optical microscopy, the relative change in capacitance ($\Delta C/C_o$) was measured, the radius of curvature ($R$) of the deformed membrane was computed, the pressure gradient (p) was determined, and an approximation that $h$ remains effectively constant (approximately 5 nm) for both coated and uncoated scenarios was made. Because the modulus changes nonlinearly for large area expansions, coated and uncoated membranes were compared using the limiting conditions of small area expansion, $E_{\parallel}^o = \lim\limits_{\Delta A/A_o \rightarrow 1} (E_{\parallel})$ .

**Area Expansion and Modulus Comparison.** Figure 3A compares the typical area expansion of uncoated bilayers to those observed for bilayers with a linked support on both sides. The original surface area of the membrane is determined using optical microscopy, where the membrane is presumed flat. As pressure increases, the measured change in relative capacitance ($\Delta C/C_o$) allows computation of the expanded surface area ($A'$). As can be seen, from 0-60 Pa applied pressure, the area expansion for coated membranes is far less than the area expansion for supported membranes. To further verify that the supportive network on the lipid bilayer provides meaningful resistance to applied stress, the statistical confidence was enhanced through replicate trials and the results are shown in Figure 8A. Three replicate trials of an uncoated bilayer and three replicate trials of a bilayer coated on two sides are shown in Fig 8A.

Following Equation 3, a plot of pressure, $p$, vs. $h(\Delta C/C_o)/R$ yields the in-plane modulus for the membrane. Assuming $h$ remains constant for both coated and uncoated membranes (e.g., 5 nm), moduli for the different membrane surfaces were directly compared. Because the modulus changes as a function of expansion, the most meaningful region for comparing moduli arises from small membrane displacements. The slopes calculated in Figure 3B show an approximate 300-fold increase in modulus $\left(E_{\parallel}^{o}\right)$ for membranes with f-actin applied to both sides, in comparison to their uncoated counterparts. See also Figure 8B, where replicate measurements of 7, 6, and 3 trials for bilayers coated on zero, one, or two sides, respectively, provide statistical confidence for the measured increase in modulus $\left(E_{\parallel}^{o}\right)$.

Previous measurements on free-standing BLMs were performed using the application of a small time-dependent alternating hydrostatic pressure [Wobschall, Journal of Colloid and Interface Science 1971, 36, 385-396; Passechnik et al., Biofizika 1973, 18, 655]. However, the magnitude of the applied pressure displaced the membrane from its equilibrium position very little. Although the instruments for these investigations differed significantly from those used herein, the moduli obtained at < 1% area expansion were typically on the order $10^6$-$10^7$ Pa. The monotonic measurement approach disclosed herein created a much larger membrane area expansion which prohibits access to moduli at expansions of < 1%. However, the values obtained for uncoated membranes are in reasonable agreement with the trend of previous measurements as the expansion approaches 1% [Hianik, T.; Passechnik, V. Bilayer Lipid Membranes: Structure and Mechanical Properties; Kluwer Academic Publishers: Norwell, MA, 1995]. This agreement validates the apparatus disclosed herein and enables comparison to a wider array of reference points for the measurement of modulus. However, this type of absolute comparison is not necessary to distinguish the overall enhancement of mechanical properties conveyed by the f-actin in the investigation performed herein.

**Accessibility of Nanopores and Membrane Electrical Resistance.** Nanopore insertion and electrical tests performed after f-actin application showed that bilayer access is not obstructed and that functional nanopores can form. Figure 4 demonstrates the typical stepwise increase in current observed. Nanopore insertion experiments involve the addition of αHL monomers in solution above the membrane. Assembly into open nanopores occurs via the normal 3 step process for αHL, where monomers: (1) adhere to the bilayer head groups; (2) diffuse within the membrane plane to form closed aggregates; and (3) unfold to pierce the membrane with the β-barrel portion of the structure. At 100 mV applied potential, membranes without αHL remain quiescent and show little current leakage (*i.e.*, a typical Gohm seal). Channel insertion produces current increases in discrete steps of approximately 125-130 pA, which is consistent with other reports for αHL in 1 M KCl.

It is noted that the short lag time between addition of αHL and the onset of channel insertion suggests the supporting f-actin framework is thin, most likely leaving large areas of the membrane directly exposed to solution. In addition, the streptavidin bridges that anchor the network to the membrane do not appear to significantly impede lateral diffusion within the plane, as monomers appear to readily adhere and aggregate to form open pores. Lastly, coated bilayers retain their typical breakdown potentials. In the absence of open nanopores, zapping the membrane with 500 mV immediately ruptures the bilayer, leaving behind an open aperture.

## Conclusion

The foregoing example describes a method for increasing the mechanical resilience of planar lipid bilayers that are commonly used for nanopore sensing applications. In some aspects, the approach involves application of a prepolymerized f-actin network that is stabilized by phalloidin and chemically linked to the bilayer through a biotin-streptavidin-biotin bridge. The data presented herein suggest this support structure is thin, consisting of a 2D network of interspersed filaments that leave a significant fraction of the bilayer widely exposed to the bathing solution. Such exposure allows quick and ready access for nanopore insertion and soluble analytes, a feature which is highly desirable for sensing applications. While the results described herein show a marked increase in membrane stability and elasticity, the quantitative increases demonstrated most likely represent the minimal enhancements that are possible with this approach. Implementation of an iterative procedure to create multiple stacked layers of f-actin that are coupled to each other through streptavidin linkages are contemplated. In this way, the thickness of the scaffold could be adjusted to an arbitrary level. This possibility is unique among the techniques for membrane support.

## Example 2

Further experiments were conducted to show that (1) the web of f-actin is attached to the unsupported bilayer; and (2) the web of actin is indeed very thin. Figure 9A depicts the typical contour of the tapered Teflon plug and Figure 9B shows a cut-away from a high-resolution confocal z stack collected at the end of the tapered Teflon plug. In this image, the bilayer is made fluorescent by incorporating fluorescently labeled lipids (*i.e.*, 5 mol% of TRITC-DHPE), rather than from fluorescently labeled actin. This image illustrates the typical location of the suspended bilayer within the tapered conduit. The lowest intensity voxels (arising from aqueous solution) have been made transparent in order to reveal the lipid location (light gray). Darker gray regions of high intensity arise from the comparatively strong photon scattering of the Teflon. Typically, lipid membranes locate at, or slightly below, the point of narrowest constriction in the tapered aperture, which is very near the top surface of the tapered conduit.

The confocal microscope produces images via sample scanning. Thus, the sample undergoes a raster movement pattern (rather than the laser beam). Individual image slices (of approximately 35 $\mu$m$^2$) can take up to 30 minutes to acquire. Indeed, the image in Figure 9B took multiple hours to acquire. Such long image acquisition times, coupled with the maximum displacement of our scanning stage (35 $\mu$m$^2$), prevents confocal images of entire membranes from being collected for many of the aperture sizes utilized. Thus, the size and number of slices acquired in confocal z stacks is typically limited to shorten experiments, or a confocal axial scan is collected through an f-actin-coated bilayer as a substitute.

Figure 10 shows a z stack of confocal images taken at 2-$\mu$m intervals from a two-sided f-actin-coated bilayer. The slice revealing individual filaments was collected in the plane of maximal fluorescent intensity. The disappearance of all filament structure at +/- 2 $\mu$m of the maximum indicates that the f-actin layer is thin (*i.e.*, below the axial resolution of confocal microscopy) and attached to the lipid membrane.

A high-resolution linear axial scan of the confocal detection volume is also shown in Figure 10 (100 nm step size) from a f-actin-coated bilayer which revealed a peak width of approximately 2 $\mu$m. This matches the peak width of the microscope's axial instrument response and further underscores the thin nature of the f-actin. Thus, the f-actin structure could be comprised of as little as a single layer, or less, of individual filaments connected to the lipid bilayer.

Individual actin filaments and actin bundles have been measured by atomic force microscopy to be 8 nm and 16 nm in diameter, respectively (Nöding et al., J. Phys. Chem. 6 122(16): 4537-4545 (2018). Thus, given an approximate 5-nm thick lipid bilayer connected to actin on two sides through biotins bound to streptavidin in opposing binding pockets (approximately 2.5 nm apart) (Le Trong et al., Acta Crystallogr D Biol Crystallogr67: 813-821 (2011)), a filament-to-filament trans-membrane distance of 26-42 nm (in localized regions of the bilayer) is estimated, if a single f-actin layer is connected to both sides. Indeed, this distance lies well-below the axial resolution of confocal microscopy.

Figure 11 shows widefield ICCD images from a two-sided f-actin-coated lipid bilayer where the illuminating laser beam has been expanded well beyond typical confocal diameters (*i.e.,* 7 $\mu$m diam.). It was found that beam expansion beyond 5-7 $\mu$m (which illuminates a greater fraction of the membrane) significantly increased the background signal and degraded the ability to detect single actin filaments. The black and white image of the aperture was acquired with the ICCD camera using bright-field illumination. Three regions of the f-actin-coated bilayer were probed in sequence using widefield laser-induced fluorescence microscopy. These regions are indicated by the overlaid circles in Figure 11. The corresponding inset images reveal the presence of individual filaments with non-fluorescent gaps between strands. These data are consistent with the hypothesis that the f-actin coating procedure creates a 2D network of filaments that covers the entirety of the lipid membrane.

**Claims**

1. A nanopore device comprising a membrane spanning an aperture, the membrane comprising:

   a lipid bilayer that is linked to a filamentous network that is external to the membrane; and
   at least one pore through the membrane; and
   wherein the filamentous network is chemically linked to the lipid bilayer, the nanopore device **characterized in that** the filamentous network is a polypeptide, an oligonucleotide, or an oligosaccharide.

2. The nanopore device of claim 1 wherein the at least one pore is an ion channel, and/or wherein the device comprises a plurality of apertures.

3. The nanopore device of claim 2, wherein:

   (a) the device comprises one pore per aperture,
   optionally
   wherein the nanopore device comprises 10, or 20, or 30, or 40, or 50, or 60, or 70, or 80, or 90, or 100, or 200, or 500, or 1000, or 2000, or 3000, or 5000, or 7000, or 10000 apertures;
   and/or
   (b) wherein the ion channel is a protein ion channel, *Staphylococcus aureus* alpha-hemolysin, *Bacillus anthracis* protective antigen 63, gramicidin, MspA (Mycobacterium smegmatis), OmpF porin, Kapton, OmpG, ClyA (Salmonella typhimurium), a non-naturally occurring compound, or derivatives thereof.

4. The nanopore device of any of claims 1-3, wherein:

(a) the chemical link is a covalent link, a non-covalent link, or an ionic link;
(b) the polypeptide is a cytoskeletal polypeptide,

optionally wherein:

(i) the cytoskeletal polypeptide is linked to the lipid bilayer through a protein bridge,
optionally
wherein the protein bridge comprises(i) biotin and streptavidin; (ii) spectrin; (iii) avidin, neutravidin, or a biotin binding protein; (iv) a bridge protein from the ERM family; (v) a bridge protein from the formin family; (vi) a transmembrane glycoprotein; or (iv) digoxygenin and an antibody directed against digoxygenin;
and/or
(ii) the cytoskeletal polypeptide is a catenin, an intermediate filament protein, a microfilament protein, or a microtubule protein

5. The nanopore device of any of claims 1 to 4, wherein the aperture is 10 nm to 1 millimeter in diameter and/or wherein the aperture is 50 microns to 500 microns in diameter,
optionally wherein:

(a) the cytoskeletal polypeptide is a catenin, an intermediate filament protein, a microfilament protein, or a microtubule protein,
optionally wherein:

(i) the catenin is alpha catenin, beta catenin, or gamma catenin;
(ii) the intermediate filament protein is desmin, glial fibrillary acidic protein, keratin, nestin, or vimentin;
(iii) the microfilament protein is actin, actinin, filamin, gelsolin, myosin, profilin, tensin, tropomyosin, troponin, or a derivative thereof;
or
(iv) the microtubule protein is dynein, tubulin, or kinesin;

and/or
(b) wherein the protein bridge comprises(i) biotin and streptavidin; (ii) spectrin; (iii) avidin, neutravidin, or a biotin binding protein; (iv) a bridge protein from the ERM family; (v) a bridge protein from the formin family; (vi) a transmembrane glycoprotein; or (iv) digoxygenin and an antibody directed against digoxygenin.

6. The nanopore device of any one of claims 1-5, further comprising a molecular motor, wherein said motor is adjacent to the at least one pore and is capable of moving a polymer with respect to the at least one pore,

optionally

wherein the molecular motor comprises a DNA polymerase, a RNA polymerase, a ribosome, an exonuclease, or a helicase and said polymer is a polynucleotide,
optionally wherein:

(a) the DNA polymerase is selected from E. coli DNA polymerase I, E. coli DNA polymerase I Large Fragment (Klenow fragment), phage T7 DNA polymerase, Phi-29 DNA polymerase, Thermus aquaticus (Taq) DNA polymerase, Thermus flavus (Tfl) DNA polymerase, Thermus Thermophilus (Tth) DNA polymerase, Thermococcus litoralis (Tli) DNA polymerase, Pyrococcus furiosus (Pfu) DNA polymerase, Bacillus stearothermophilus (Bst) DNA polymerase, AMV reverse transcriptase, MMLV reverse transcriptase, and HIV-1 reverse transcriptase;
(b) the RNA polymerase is selected from T7 RNA polymerase, T3 RNA polymerase, SP6 RNA polymerase, and E. coli RNA polymerase;
or
(c) the exonuclease is selected from exonuclease Lambda, T7 Exonuclease, Exo III, RecJ, Exonuclease, Exo I, and Exo T;
and/or
(d) the helicase is selected from E-coli bacteriophage T7 gp4 and T4 gp41 gene proteins, E. coli protein DnaB, E. coli protein RuvB, and E. coli protein rho.

and/or
wherein the lipid bilayer comprises a plurality of lipid groups comprising one or more of diphytanoyl 1,2,-diacyl-sn-glycero-3-[phosphor-L-serine] (DiPHyPC), 1,2-dioleoyl-sn-glycero-3-phosphocholine (DOPC), 1,2-dimyristoyl-sn-phosphatidylcholine (DMPC), 1-palmitoyl-2-oleoyl-sn-phosphatidylcholine (POPC), 1,2-distearoyl-sn-glycero-3-phospho-(1'-rac-glycerol) (DSPG), 1,2-dioleoyl-sn-glycero-3-phospho-(1'-rac-glycerol) (DOPG), 1,2-distearoyl-sn-glycero-3-phosphocholine (DSPC), 1,2-dipalmitoyl-sn-glycero-3-phosphocholine (DPPC), 1,2-di-(9Z-octadecenoyl)-sn-glycero-3-phosphoethanolamine (DOPE), and 1,2-dihexadecanoyl-sn-glycero-3-phosphoethanolamine (DPPE).

7. A method of analyzing a target polymer comprising contacting the target polymer to the nanopore device of claim 1 to allow the target polymer to move with respect to the at least one pore to produce a signal, and monitoring the signal corresponding to the movement of the target polymer with respect to the pore, thereby analyzing the target polymer.

8. The method of claim 7, wherein the signal monitoring comprises measuring a monomer-dependent characteristic of the target polymer while the target polymer moves with respect to the pore,
optionally
wherein the monomer dependent property is the identity of a monomer or the number of monomers in the polymer.

9. The method of either of claims 7 or 8:

(a) further comprising altering the rate of movement of the polymer before, during, or after the signal monitoring;
and/or
(b) wherein the target polymer is an oligonucleotide, a polypeptide, or an oligosaccharide,
optionally
wherein the oligonucleotide is DNA;
and/or
(c) wherein the analyzing comprises a chemical characterization,
optionally

wherein the chemical characterization is a characterization of DNA, a synthetic polymer, a small molecule, or an ion,
optionally
wherein the characterization of DNA comprises nucleotide sequencing or genotyping.

10. A method of forming a nanopore device, the method comprising:

providing a membrane having a thickness and spanning an aperture, said membrane comprising a lipid bilayer;
associating the membrane with at least one pore such that the at least one pore extends through the membrane over the thickness of the membrane, thus forming at least one channel connecting a first side and a second side of the membrane, wherein the pore has a first opening that opens to the first side of the membrane, a second opening that opens to the second side of the membrane, and a depth; and
associating the membrane comprising at least one pore with a filamentous network that links to the first side of the membrane and is external to the membrane, thus forming the nanopore device, wherein the filamentous network is chemically linked to the membrane,
the method **characterized in that** the filamentous network is a polypeptide, an oligonucleotide, or an oligosaccharide.

11. The method of claim 10, wherein the chemical link is a covalent link, a non-covalent link, or an ionic link.

12. The method of either of claims 10 or 11, wherein the polypeptide is a cytoskeletal polypeptide,
optionally wherein:

(i) the cytoskeletal polypeptide is linked to the membrane through a protein bridge,
optionally
wherein the protein bridge comprises (i) biotin and streptavidin; (ii) spectrin; (iii) avidin, neutravidin, or a biotin binding protein; (iv) a bridge protein from the ERM family; (v) a bridge protein from the formin family; (vi) a transmembrane glycoprotein; or (iv) digoxygenin and an antibody directed against digoxygenin;

and/or
(ii) wherein the cytoskeletal polypeptide is a catenin, an intermediate filament protein, a microfilament protein, or a microtubule protein.

13. The method of any one of claims 10 to 12, wherein the aperture is from 10 nm to 1 millimeter in diameter and/or wherein the aperture is from 50 microns to 500 microns in diameter, optionally wherein:

(a) the cytoskeletal polypeptide is a catenin, an intermediate filament protein, a microfilament protein, or a microtubule protein, optionally wherein:

(i) the catenin is alpha catenin, beta catenin, or gamma catenin;
(ii) the intermediate filament protein is desmin, glial fibrillary acidic protein, keratin, nestin, or vimentin;
(iii) the microfilament protein is actin, actin-related protein, actinin, filamin, gelsolin, myosin, profilin, tensin, tropomyosin, or troponin;
or
(iv) wherein the microtubule protein is dynein, tubulin, or kinesin;

and/or
(b) wherein the protein bridge comprises (i) biotin and streptavidin; (ii) spectrin; (iii) avidin, neutravidin, or a biotin binding protein; (iv) a bridge protein from the ERM family; (v) a bridge protein from the formin family; (vi) a transmembrane glycoprotein; or (iv) digoxygenin and an antibody directed against digoxygenin.

14. The method of any one of claims 10-13, further comprising a molecular motor, wherein said motor is adjacent to the at least one pore and is capable of moving a polymer with respect to the at least one pore, optionally

wherein the molecular motor comprises a DNA polymerase, a RNA polymerase, a ribosome, an exonuclease, or a helicase and said polymer is a polynucleotide, optionally wherein:

(a) the DNA polymerase is selected from E. coli DNA polymerase I, E. coli DNA polymerase I Large Fragment (Klenow fragment), phage T7 DNA polymerase, Phi-29 DNA polymerase, Thermus aquaticus (Taq) DNA polymerase, Thermus flavus (Tfl) DNA polymerase, Thermus Thermophilus (Tth) DNA polymerase, Thermococcus litoralis (Tli) DNA polymerase, Pyrococcus furiosus (Pfu) DNA polymerase, Bacillus stearothermophilus (Bst) DNA polymerase, AMV reverse transcriptase, MMLV reverse transcriptase, and HIV-1 reverse transcriptase;
(b) the RNA polymerase is selected from T7 RNA polymerase, T3 RNA polymerase, SP6 RNA polymerase, and E. coli RNA polymerase;
(c) the exonuclease is selected from exonuclease Lambda, T7 Exonuclease, Exo III, RecJ, Exonuclease, Exo I, and Exo T;
or
(d) the helicase is selected from E-coli bacteriophage T7 gp4 and T4 gp41 gene proteins, E. coli protein DnaB, E. coli protein RuvB, and E. coli protein rho.

15. The method of any one of claims 10-14, wherein:

(a) the at least one pore is an ion channel, optionally wherein the ion channel is a protein ion channel, Staphylococcus aureus alpha-hemolysin, Bacillus anthracis protective antigen 63, gramicidin, MspA (Mycobacterium smegmatis), OmpF porin, Kapton, OmpG, ClyA (Salmonella typhimurium), or a non-naturally occurring compound;
(b) the lipid bilayer comprises a plurality of lipid groups comprising diphytanoyl 1,2,-diacyl-sn-glycero-3-[phosphor-L-serine] (DiPHyPC), 1,2-dioleoyl-sn-glycero-3-phosphocholine (DOPC), 1,2-dimyristoyl-sn-phosphatidylcholine (DMPC), 1-palmitoyl-2-oleoyl-sn-phosphatidylcholine (POPC), 1,2-distearoyl-sn-glycero-3-phospho-(1'-rac-glycerol) (DSPG), 1,2-dioleoyl-sn-glycero-3-phospho-(1'-rac-glycerol) (DOPG), 1,2-distearoyl-sn-glycero-3-phosphocholine (DSPC), 1,2-dipalmitoyl-sn-glycero-3-phosphocholine (DPPC), 1,2-di-(9Z-octadecenoyl)-sn-glycero-3-phosphoethanolamine (DOPE), or 1,2-dihexadecanoyl-sn-glycero-3-phosphoethanolamine (DPPE).

**Patentansprüche**

1. Nanoporenvorrichtung, umfassend eine Membran, die eine Öffnung überspannt, wobei die Membran Folgendes umfasst:

   eine Lipiddoppelschicht, die mit einem fadenförmigen Netzwerk verbunden ist, das sich außerhalb der Membran befindet; und
   mindestens eine Pore durch die Membran; und
   wobei das fadenförmige Netzwerk chemisch an die Lipiddoppelschicht gebunden ist, wobei die Nanoporenvorrichtung **dadurch gekennzeichnet ist, dass** das fadenförmige Netzwerk ein Polypeptid, ein Oligonukleotid oder ein Oligosaccharid ist.

2. Nanoporenvorrichtung nach Anspruch 1, wobei die mindestens eine Pore ein Ionenkanal ist, und/oder wobei die Vorrichtung eine Vielzahl von Öffnungen umfasst.

3. Nanoporenvorrichtung nach Anspruch 2, wobei:

   (a) die Vorrichtung eine Pore pro Öffnung umfasst, optional
   wobei die Nanoporenvorrichtung 10 oder 20 oder 30 oder 40 oder 50 oder 60 oder 70 oder 80 oder 90 oder 100 oder 200 oder 500 oder 1000 oder 2000 oder 3000 umfasst, oder 5000 oder 7000 oder 10000 Öffnungen umfasst;
   und/oder
   (b) wobei der Ionenkanal ein Protein-Ionenkanal, *Staphylococcus aureus* alpha-Hämolysin, *Bacillus anthracis* Schutzantigen 63, Gramicidin, MspA (Mycobacterium smegmatis), OmpF-Porin, Kapton, OmpG, ClyA (Salmonella typhimurium), eine nicht natürlich vorkommende Verbindung, oder Derivate davon ist.

4. Nanoporenvorrichtung nach einem der Ansprüche 1 bis 3, wobei:

   (a) die chemische Bindung eine kovalente Bindung, eine nicht-kovalente Bindung oder eine ionische Bindung ist;
   (b) das Polypeptid ein Zytoskelett-Polypeptid ist, optional wobei:

   (i) das Zytoskelett-Polypeptid über eine Proteinbrücke mit der Lipiddoppelschicht verbunden ist, optional
   wobei die Proteinbrücke (i) Biotin und Streptavidin;
   (ii) Spektrin; (iii) Avidin, Neutravidin oder ein Biotin-bindendes Protein; (iv) ein Brückenprotein aus der ERM-Familie; (v) ein Brückenprotein aus der Formin-Familie; (vi) ein Transmembran-Glykoprotein; oder (iv) Digoxigenin und einen gegen Digoxigenin gerichteten Antikörper umfasst;
   und/oder
   (ii) das Zytoskelett-Polypeptid ein Catenin, ein intermediäres Filamentprotein, ein Mikrofilamentprotein oder ein Mikrotubuliprotein ist.

5. Nanoporenvorrichtung nach einem der Ansprüche 1 bis 4, wobei die Öffnung einen Durchmesser von 10 nm bis 1 Millimeter aufweist, und/oder wobei die Öffnung einen Durchmesser von 50 Mikrometer bis 500 Mikrometer aufweist, optional wobei:

   (a) das Zytoskelett-Polypeptid ein Catenin, ein intermediäres Filamentprotein, ein Mikrofilamentprotein oder ein Mikrotubuliprotein ist,
   optional wobei:

   (i) das Catenin Alpha-Catenin, Beta-Catenin oder Gamma-Catenin ist;
   (ii) das intermediäre Filamentprotein Desmin, saures Gliafibrillenprotein, Keratin, Nestin oder Vimentin ist;
   (iii) das Mikrofilamentprotein Actin, Actinin, Filamin, Gelsolin, Myosin, Profilin, Tensin, Tropomyosin, Troponin oder ein Derivat davon ist;
   oder

(iv) das Mikrotubuli-Protein Dynein, Tubulin oder Kinesin ist;

und/oder
(b) wobei die Proteinbrücke (i) Biotin und Streptavidin; (ii) Spektrin; (iii) Avidin, Neutravidin oder ein Biotin-bindendes Protein; (iv) ein Brückenprotein aus der ERM-Familie; (v) ein Brückenprotein aus der Formin-Familie; (vi) ein Transmembran-Glykoprotein; oder (iv) Digoxigenin und einen gegen Digoxigenin gerichteten Antikörper umfasst.

6. Nanoporenvorrichtung nach einem der Ansprüche 1 bis 5, ferner umfassend einen Molekularmotor, wobei der Motor benachbart zu der mindestens einen Pore ist und in der Lage ist, ein Polymer in Bezug auf die mindestens eine Pore zu bewegen,
optional
wobei der molekulare Motor eine DNA-Polymerase, eine RNA-Polymerase, ein Ribosom, eine Exonuklease oder eine Helikase umfasst und das Polymer ein Polynukleotid ist, optional wobei:

(a) die DNA-Polymerase ausgewählt ist aus E. coli-DNA-Polymerase I, E. coli-DNA-Polymerase I-Großfragment (Klenow-Fragment), Phagen-T7-DNA-Polymerase, Phi-29-DNA-Polymerase, Thermus aquaticus (Taq) DNA-Polymerase, Thermus flavus (Tfl) DNA-Polymerase, Thermus Thermophilus (Tth) DNA-Polymerase, Thermococcus litoralis (Tli) DNA-Polymerase, Pyrococcus furiosus (Pfu)-DNA-Polymerase, Bacillus stearothermophilus (Bst)-DNA-Polymerase, AMV-Reverse-Transkriptase, MMLV-Reverse-Transkriptase und HIV-1-Reverse-Transkriptase;
(b) die RNA-Polymerase ausgewählt ist aus T7-RNA-Polymerase, T3-RNA-Polymerase, SP6-RNA-Polymerase und E. coli-RNA-Polymerase;
oder
(c) die Exonuklease ausgewählt ist aus Exonuklease Lambda, T7 Exonuklease, Exo III, RecJ$_1$ Exonuklease, Exo I und Exo T;
und/oder
(d) die Helikase ausgewählt ist aus E. coli-Bakteriophagen T7 gp4 und T4 gp41-Genproteinen, E. coli-Protein DnaB, E. coli-Protein RuvB und E. coli-Protein rho.

und/oder
wobei die Lipiddoppelschicht eine Vielzahl von Lipidgruppen umfasst, umfassend eine oder mehrere von Diphytanoyl 1,2,-diacyl-sn-glycero-3-[phosphor-L-serin] (DiPHyPC), 1,2-Dioleoyl-sn-Glycero-3-Phosphocholin (DOPC), 1,2-Dimyristoyl-sn-phosphatidylcholin (DMPC), 1-Palmitoyl-2-oleoyl-sn-phosphatidylcholin (POPC), 1,2-Distearoyl-sn-glycero-3-phospho-(1'-rac-glycerin) (DSPG), 1,2-Dioleoyl-sn-glycero-3-phospho-(1'-rac-glycerin) (DOPG), 1,2-Distearoyl-sn-glycero-3-phosphocholin (DSPC), 1,2-Dipalmitoyl-sn-glycero-3-phosphocholin (DPPC), 1,2-Di-(9Z-octadecenoyl)-sn-glycero-3-phosphoethanolamin (DOPE) und 1,2-Dihexadecanoyl-sn-glycero-3-phosphoethanolamin (DPPE).

7. Verfahren zum Analysieren eines Zielpolymers, umfassend das Inkontaktbringen des Zielpolymers mit der Nanoporenvorrichtung nach Anspruch 1, um zu ermöglichen, dass sich das Zielpolymer in Bezug auf die mindestens eine Pore bewegt, um ein Signal zu erzeugen, und Überwachen des Signals, das der Bewegung des Zielpolymers in Bezug auf die Pore entspricht, wodurch das Zielpolymer analysiert wird.

8. Verfahren nach Anspruch 7, wobei die Signalüberwachung das Messen einer monomerabhängigen Eigenschaft des Zielpolymers umfasst, während sich das Zielpolymer in Bezug auf die Pore bewegt,
optional
wobei die monomerabhängige Eigenschaft die Identität eines Monomers oder die Anzahl von Monomeren in dem Polymer ist.

9. Verfahren nach Anspruch 7 oder 8:

(a) ferner umfassend das Ändern der Bewegungsgeschwindigkeit des Polymers vor, während oder nach der Signalüberwachung;
und/oder
(b) wobei das Zielpolymer ein Oligonukleotid, ein Polypeptid oder ein Oligosaccharid ist,

optional

wobei das Oligonukleotid DNA ist;

und/oder
(c) wobei das Analysieren eine chemische Charakterisierung umfasst,

optional
wobei die chemische Charakterisierung eine Charakterisierung von DNA, einem synthetischen Polymer, einem kleinen Molekül oder einem Ion ist,
optional
wobei die Charakterisierung von DNA Nukleotidsequenzierung oder Genotypisierung umfasst.

10. Verfahren zum Bilden einer Nanoporenvorrichtung, wobei das Verfahren Folgendes umfasst:

Bereitstellen einer Membran, die eine Dicke aufweist und eine Öffnung überspannt, wobei die Membran eine Lipiddoppelschicht umfasst;
Zuordnen der Membran zu mindestens einer Pore, so dass sich die mindestens eine Pore durch die Membran über die Dicke der Membran erstreckt und somit mindestens einen Kanal bildet, der eine erste Seite und eine zweite Seite der Membran verbindet, wobei die Pore eine erste Öffnung, die sich zur ersten Seite der Membran öffnet, eine zweite Öffnung, die sich zur zweiten Seite der Membran öffnet, und eine Tiefe aufweist; und
Zuordnen der Membran, die mindestens eine Pore umfasst, mit einem fadenförmigen Netzwerk, das mit der ersten Seite der Membran verbunden ist und sich außerhalb der Membran befindet, wodurch die Nanoporenvorrichtung gebildet wird, wobei das fadenförmige Netzwerk chemisch mit der Membran verbunden ist, wobei das Verfahren **dadurch gekennzeichnet ist, dass** das fadenförmige Netzwerk ein Polypeptid, ein Oligonukleotid oder ein Oligosaccharid ist.

11. Verfahren nach Anspruch 10, wobei die chemische Bindung eine kovalente Bindung, eine nicht-kovalente Bindung oder eine ionische Bindung ist.

12. Verfahren nach Anspruch 10 oder 11, wobei das Polypeptid ein Zytoskelett-Polypeptid ist, optional wobei:

(i) das Zytoskelett-Polypeptid über eine Proteinbrücke mit der Membran verbunden ist,
optional
wobei die Proteinbrücke (i) Biotin und Streptavidin;
(ii) Spektrin; (iii) Avidin, Neutravidin oder ein Biotin-bindendes Protein; (iv) ein Brückenprotein aus der ERM-Familie; (v) ein Brückenprotein aus der Formin-Familie; (vi) ein Transmembran-Glykoprotein; oder (iv) Digoxigenin und einen gegen Digoxigenin gerichteten Antikörper umfasst;
und/oder
(ii) wobei das Cytoskelett-Polypeptid ein Catenin, ein intermediäres Filamentprotein, ein Mikrofilamentprotein oder ein Mikrotubuliprotein ist.

13. Verfahren nach einem der Ansprüche 10 bis 12, wobei die Öffnung einen Durchmesser von 10 nm bis 1 Millimeter aufweist und/oder wobei die Öffnung einen Durchmesser von 50 Mikrometer bis 500 Mikrometer aufweist, optional wobei:

(a) das Zytoskelett-Polypeptid ein Catenin, ein intermediäres Filamentprotein, ein Mikrofilamentprotein oder ein Mikrotubuliprotein ist,
optional wobei:

(i) das Catenin Alpha-Catenin, Beta-Catenin oder Gamma-Catenin ist;
(ii) das intermediäre Filamentprotein Desmin, saures Gliafibrillenprotein, Keratin, Nestin oder Vimentin ist;
(iii) das Mikrofilamentprotein Aktin, mit Aktin verwandtes Protein, Aktinin, Filamin, Gelsolin, Myosin, Profilin, Tensin, Tropomyosin oder Troponin ist;
oder
(iv) wobei das Mikrotubuli-Protein Dynein, Tubulin oder Kinesin ist;
und/oder

(b) wobei die Proteinbrücke (i) Biotin und Streptavidin; (ii) Spektrin; (iii) Avidin, Neutravidin oder ein Biotin-bindendes Protein; (iv) ein Brückenprotein aus der ERM-Familie; (v) ein Brückenprotein aus der Formin-Familie; (vi) ein Transmembran-Glykoprotein; oder (iv) Digoxigenin und einen gegen Digoxigenin gerichteten Antikörper umfasst.

**14.** Verfahren nach einem der Ansprüche 10 bis 13, ferner umfassend einen molekularen Motor,

wobei der Motor benachbart zu der mindestens einen Pore ist und in der Lage ist, ein Polymer in Bezug auf die mindestens eine Pore zu bewegen,
optional
wobei der molekulare Motor eine DNA-Polymerase, eine RNA-Polymerase, ein Ribosom, eine Exonuklease oder eine Helikase umfasst und das Polymer ein Polynukleotid ist, optional wobei:

(a) die DNA-Polymerase ausgewählt ist aus E. coli-DNA-Polymerase I, E. coli-DNA-Polymerase I-Großfragment (Klenow-Fragment), Phagen-T7-DNA-Polymerase, Phi-29-DNA-Polymerase, Thermus aquaticus (Taq)-DNA-Polymerase, Thermus flavus (Tfl)-DNA-Polymerase, Thermus Thermophilus (Tth) DNA-Polymerase, Thermococcus litoralis (Tli) DNA-Polymerase, DNA-Polymerase von Pyrococcus furiosus (Pfu), DNA-Polymerase von Bacillus stearothermophilus (Bst), AMV-Reverse-Transkriptase, MMLV-Reverse-Transkriptase und HIV-1-Reverse-Transkriptase;
(b) die RNA-Polymerase ausgewählt ist aus T7-RNA-Polymerase, T3-RNA-Polymerase, SP6-RNA-Polymerase und E. coli-RNA-Polymerase;
(c) die Exonuklease ausgewählt ist aus Exonuklease Lambda, T7 Exonuklease, Exo III, RecJi Exonuklease, Exo I und Exo T;
oder
(d) die Helikase ausgewählt ist aus E. coli-Bakteriophagen T7 gp4 und T4 gp41-Genproteinen, E. coli-Protein DnaB, E. coli-Protein RuvB und E. coli-Protein rho.

**15.** Verfahren nach einem der Ansprüche 10-14, wobei:

(a) die mindestens eine Pore ein Ionenkanal ist, optional
wobei der Ionenkanal ein Proteinionenkanal, *Staphylococcus aureus* alpha-Hämolysin, *Bacillus anthracis* Schutzantigen 63, Gramicidin, MspA (Mycobacterium smegmatis), OmpF-Porin, Kapton, OmpG, ClyA (Salmonella typhimurium) oder eine nicht natürlich vorkommende Verbindung ist;
(b) die Lipiddoppelschicht eine Vielzahl von Lipidgruppen umfasst, die Diphytanoyl-1,2-diacyl-sn-glycero-3-[phosphor-L-serin] (DiPHyPC), 1,2-Dioleoyl-sn-Glycero-3-Phosphocholin (DOPC), 1,2-Dimyristoyl-snphosphatidylcholin (DMPC), 1-Palmitoyl-2-oleoyl-sn-phosphatidylcholin (POPC), 1,2-Distearoyl-sn-glycero-3-phospho-(1'-rac-glycerin) (DSPG), 1,2-Dioleoyl-sn-glycero-3-phospho-(1'-rac-glycerin) (DOPG), 1,2-Distearoyl-sn-glycero-3-phosphocholin (DSPC), 1,2-Dipalmitoyl-sn-glycero-3-phosphocholin (DPPC), 1,2-Di-(9Z-octadecenoyl)-sn-glycero-3-phosphoethanolamin (DOPE) oder 1,2-Dihexadecanoyl-sn-glycero-3-phosphoethanolamin (DPPE) umfassen.

## Revendications

**1.** Dispositif à nanopores comprenant une membrane couvrant une ouverture, la membrane comprenant :

une bicouche lipidique qui est liée à un réseau filamenteux qui est externe à la membrane ; et
au moins un pore à travers la membrane ; et
dans lequel le réseau filamenteux est lié chimiquement à la bicouche lipidique, le dispositif à nanopores étant **caractérisé en ce que** le réseau filamenteux est un polypeptide, un oligonucléotide ou un oligosaccharide.

**2.** Dispositif à nanopores selon la revendication 1, dans lequel l'au moins un pore est un canal ionique, et/ou dans lequel le dispositif comprend une pluralité d'ouvertures.

**3.** Dispositif à nanopores selon la revendication 2, dans lequel :

(a) le dispositif comprend un pore par ouverture, éventuellement
dans lequel le dispositif à nanopores comprend 10, ou 20, ou 30, ou 40, ou 50, ou 60, ou 70, ou 80, ou 90, ou 100, ou 200, ou 500, ou 1 000, ou 2 000, ou 3 000, ou 5 000, ou 7 000, ou 10 000 ouvertures ;

et/ou

(b) dans lequel le canal ionique est un canal ionique protéique, l'alpha-hémolysine de *Staphylococcus aureus,* l'antigène protecteur 63 de *Bacillus anthracis,* la gramicidine, MspA (Mycobacterium smegmatis), la porine OmpF, le Kapton, OmpG, ClyA (Salmonella typhimurium), un composé d'origine non naturelle, ou des dérivés de ceux-ci.

4. Dispositif à nanopores selon l'une quelconque des revendications 1 à 3, dans lequel :

(a) la liaison chimique est une liaison covalente, une liaison non covalente ou une liaison ionique ;
(b) le polypeptide est un polypeptide cytosquelettique,
éventuellement dans lequel :

(i) le polypeptide cytosquelettique est lié à la bicouche lipidique à travers un pont protéique, éventuellement
dans lequel le pont protéique comprend (i) de la biotine et de la streptavidine ; (ii) de la spectrine ; (iii) de l'avidine, de la neutravidine ou une protéine de liaison à la biotine ; (iv) une protéine-pont de la famille ERM ; (v) une protéine-pont de la famille des termines ; (vi) une glycoprotéine transmembranaire ; ou (iv) la digoxygénine et un anticorps dirigé contre la digoxygénine ;
et/ou
(ii) le polypeptide cytosquelettique est une caténine, une protéine de filament intermédiaire, une protéine de microfilament ou une protéine microtubulaire.

5. Dispositif à nanopores selon l'une quelconque des revendications 1 à 4, dans lequel l'ouverture a un diamètre de 10 nm à 1 millimètre et/ou dans lequel l'ouverture a un diamètre de 50 microns à 500 microns, éventuellement dans lequel :

(a) le polypeptide cytosquelettique est une caténine, une protéine de filament intermédiaire, une protéine de microfilament ou une protéine microtubulaire, éventuellement dans lequel :

(i) la caténine est l'alpha-caténine, la bêta-caténine ou la gamma-caténine ;
(ii) la protéine de filament intermédiaire est la desmine, la protéine acide fibrillaire gliale, la kératine, la nestine ou la vimentine ;
(iii) la protéine de microfilament est l'actine, l'actinine, la filamine, la gelsoline, la myosine, la profiline, la tensine, la tropomyosine, la troponine ou un dérivé de celles-ci ;
ou
(iv) la protéine microtubulaire est la dynéine, la tubuline ou la kinésine ;

et/ou
(b) dans lequel le pont protéique comprend (i) de la biotine et de la streptavidine ; (ii) de la spectrine ; (iii) de l'avidine, de la neutravidine ou une protéine de liaison à la biotine ; (iv) une protéine-pont de la famille ERM ; (v) une protéine-pont de de la famille des formines ; (vi) une glycoprotéine transmembranaire ; ou (iv) la digoxygénine et un anticorps dirigé contre la digoxygénine.

6. Dispositif à nanopores selon l'une quelconque des revendications 1 à 5, comprenant en outre un moteur moléculaire, dans lequel ledit moteur est adjacent à l'au moins un pore et est capable de déplacer un polymère par rapport à l'au moins un pore,

éventuellement

dans lequel le moteur moléculaire comprend une ADN polymérase, une ARN polymérase, un ribosome, une exonucléase ou une hélicase et ledit polymère est un polynucléotide,
éventuellement dans lequel :

(a) l'ADN polymérase est choisie parmi l'ADN polymérase I de E. coli, l'ADN polymérase I, grand fragment, de E. coli (fragment de Klenow), l'ADN polymérase de phage T7, l'ADN polymérase de Phi-29, l'ADN polymérase de Thermus aquaticus (Taq), l'ADN polymérase de Thermus flavus (Tfl), l'ADN polymérase de Thermus Thermophilus (Tth), l'ADN polymérase de Thermococcus litoralis (Tli), ADN polymérase de Pyrococcus furiosus (Pfu), l'ADN polymérase de Bacillus stearothermophilus (Bst), la Transcriptase inverse AMV, la transcriptase inverse MMLV et la transcriptase inverse VIH-1 ;
(b) l'ARN polymérase est choisie parmi l'ARN polymérase T7, l'ARN polymérase T3, l'ARN polymérase SP6 et l'ARN polymérase de E. coli ;
ou
(c) l'exonucléase est choisie parmi l'exonucléase Lambda, l'exonucléase T7, Exo III, l'exonucléase RecJi, Exo I et Exo T ; et/ou
(d) l'hélicase est choisie parmi les protéines des gènes T7 gp4 et T4 gp41 du bactériophage E. coli, la protéine DnaB de E. coli, la protéine RuvB de E. coli et la protéine rho de E. coli.

et/ou

dans lequel la bicouche lipidique comprend une pluralité de groupes lipidiques comprenant un ou plusieurs parmi la 1,2,-diacyl-sn-glycéro-3-[phosphor-L-sérine] de diphytanoyle (DiPHyPC), la 1,2-dioléoyl-sn-glycéro-3-phosphocholine (DOPC), la 1,2-dimyristoyl-sn-phosphatidylcholine (DMPC), la 1-palmitoyl-2-oléoyl-sn-phosphatidylcholine (POPC), le 1,2-distéaroyl-sn-glycéro-3-phospho-(1'-rac-glycérol) (DSPG), le 1,2-dioléoyl-sn-glycéro-3-phospho-(1'-rac-glycérol) (DOPG), la 1,2-distéaroyl-sn-glycéro-3-phosphocholine (DSPC), la 1,2-dipalmitoyl-sn-glycéro-3-phosphocholine (DPPC), la 1,2-di-(9Z-octadécénoyl)-sn-glycéro-3-phosphoéthanolamine (DOPE) et la 1,2-dihexadécanoyl-sn-glycéro-3-phosphoéthanolamine (DPPE) .

7. Procédé d'analyse d'un polymère cible comprenant la mise en contact du polymère cible avec le dispositif à nanopores selon la revendication 1 pour permettre au polymère cible de se déplacer par rapport à l'au moins un pore pour produire un signal, et la surveillance du signal correspondant au mouvement du polymère cible par rapport au pore, analysant ainsi le polymère cible.

8. Procédé selon la revendication 7, dans lequel la surveillance de signal comprend la mesure d'une caractéristique dépendante du monomère du polymère cible tandis que le polymère cible se déplace par rapport au pore, éventuellement
dans lequel la propriété dépendante du monomère est l'identité d'un monomère ou le nombre de monomères dans le polymère.

9. Procédé selon l'une des revendications 7 ou 8 :

(a) comprenant en outre la modification de la vitesse de déplacement du polymère avant, pendant ou après la surveillance de signal ;
et/ou
(b) dans lequel le polymère cible est un oligonucléotide, un polypeptide ou un oligosaccharide, éventuellement
dans lequel l'oligonucléotide est l'ADN ;
et/ou
(c) dans lequel l'analyse comprend une caractérisation chimique, éventuellement

dans lequel la caractérisation chimique est une caractérisation d'ADN, d'un polymère synthétique, d'une petite molécule ou d'un ion,
éventuellement
dans lequel la caractérisation d'ADN comprend le séquençage ou le génotypage nucléotidique.

10. Procédé de formation d'un dispositif à nanopores, le procédé comprenant :

la fourniture d'une membrane ayant une épaisseur et s'étendant sur une ouverture, ladite membrane comprenant une bicouche lipidique ;
l'association de la membrane à au moins un pore de sorte que l'au moins un pore s'étend à travers la membrane sur l'épaisseur de la membrane, formant ainsi au moins un canal connectant un premier côté et un second côté de la membrane, dans lequel le pore a une première ouverture qui s'ouvre sur le premier côté de la membrane, une seconde ouverture qui s'ouvre sur le second côté de la membrane, et une profondeur ; et
l'association de la membrane comprenant au moins un pore avec un réseau filamenteux relié au premier côté de la membrane et extérieur à la membrane, formant ainsi le dispositif à nanopores, dans lequel le réseau filamenteux est lié chimiquement à la membrane,

le procédé étant **caractérisé en ce que** le réseau filamenteux est un polypeptide, un oligonucléotide ou un oligosaccharide.

11. Procédé selon la revendication 10, dans lequel la liaison chimique est une liaison covalente, une non covalente ou une liaison ionique.

12. Procédé selon l'une des revendications 10 ou 11, dans lequel le polypeptide est un polypeptide cytosquelettique, éventuellement dans lequel :

   (i) le polypeptide cytosquelettique est lié à la membrane à travers un pont protéique, éventuellement
   dans lequel le pont protéique comprend

   (i) de la biotine et de la streptavidine ;
   (ii) de la spectrine ; (iii) de l'avidine, de la neutravidine ou une protéine de liaison à la biotine ; (iv) une protéine-pont de la famille ERM ; (v) une protéine-pont de la famille des formines ; (vi) une glycoprotéine transmembranaire ; ou (iv) la digoxygénine et un anticorps dirigé contre la digoxygénine ;

   et/ou
   (ii) dans lequel le polypeptide cytosquelettique est une caténine, une protéine de filament intermédiaire, une protéine de microfilament ou une protéine microtubulaire,

13. Procédé selon l'une quelconque des revendications 10 à 12, dans lequel l'ouverture a un diamètre de 10 nm à 1 millimètre et/ou dans lequel l'ouverture a un diamètre de 50 microns à 500 microns, éventuellement dans lequel :

   (a) le polypeptide cytosquelettique est une caténine, une protéine de filament intermédiaire, une protéine de microfilament ou une protéine microtubulaire, éventuellement dans lequel :

   (i) la caténine est l'alpha-caténine, la bêta-caténine ou la gamma-caténine ;
   (ii) la protéine de filament intermédiaire est la desmine, la protéine acide fibrillaire gliale, la kératine, la nestine ou la vimentine ;
   (iii) la protéine de microfilament est l'actine, la protéine apparentée à l'actine, l'actinine, la filamine, la gelsoline, la myosine, la profiline, la tensine, la tropomyosine ou la troponine ;
   ou
   (iv) dans lequel la protéine microtubulaire est la dynéine, la tubuline ou la kinésine ;

   et/ou
   (b) dans lequel le pont protéique comprend (i) de la biotine et de la streptavidine ; (ii) de la spectrine ; (iii) de l'avidine, de la neutravidine ou une protéine de liaison à la biotine ; (iv) une protéine-pont de la famille ERM ; (v) une protéine-pont de la famille des termines ; (vi) une glycoprotéine transmembranaire ; ou (iv) la digoxygénine et un anticorps dirigé contre la digoxygénine.

14. Dispositif à nanopores selon l'une quelconque des revendications 10 à 13, comprenant en outre un moteur moléculaire, dans lequel ledit moteur est adjacent à l'au moins un pore et est capable de déplacer un polymère par rapport à l'au moins un pore, éventuellement

   dans lequel le moteur moléculaire comprend une ADN polymérase, une ARN polymérase, un ribosome, une exonucléase ou une hélicase et ledit polymère est un polynucléotide, éventuellement dans lequel :

   (a) l'ADN polymérase est choisie parmi l'ADN polymérase I de E. coli, l'ADN polymérase I, grand fragment, de E. coli (fragment de Klenow), l'ADN polymérase de phage T7, l'ADN polymérase de Phi-29, l'ADN polymérase de Thermus aquaticus (Taq), l'ADN polymérase de Thermus flavus (Tfl), l'ADN polymérase de Thermus Thermophilus (Tth), l'ADN polymérase de Thermococcus litoralis (Tli), ADN polymérase de Pyrococcus furiosus (Pfu), l'ADN polymérase de Bacillus stearothermophilus (Bst), la Transcriptase inverse AMV, la transcriptase inverse MMLV et la transcriptase inverse VIH-1 ;

(b) l'ARN polymérase est choisie parmi l'ARN polymérase T7, l'ARN polymérase T3, l'ARN polymérase SP6 et l'ARN polymérase de E. coli ;

(c) l'exonucléase est choisie parmi l'exonucléase Lambda, l'exonucléase T7, Exo III, l'exonucléase RecJi, Exo I et Exo T ; ou

(d) l'hélicase est choisie parmi les protéines des gènes T7 gp4 et T4 gp41 du bactériophage E. coli, la protéine DnaB de E. coli, la protéine RuvB de E. coli et la protéine rho de E. coli.

**15.** Procédé selon l'une quelconque des revendications 10 à 14, dans lequel :

(a) l'au moins un pore est un canal ionique, éventuellement

dans lequel le canal ionique est un canal ionique protéique, l'alpha-hémolysine de *Staphylococcus aureus*, l'antigène protecteur 63 de *Bacillus anthracis*, la gramicidine, MspA (Mycobacterium smegmatis), la porine OmpF, le Kapton, OmpG, ClyA (Salmonella typhimurium) ou un composé d'origine non naturelle.

(b) la bicouche lipidique comprend une pluralité de groupes lipidiques comprenant la 1,2,-diacyl-sn-glycéro-3-[phosphor-L-sérine] de diphytanoyle (DiPHyPC), la 1,2-dioléoyl-sn-glycéro-3-phosphocholine (DOPC), la 1,2-dimyristoyl-sn-phosphatidylcholine (DMPC), la 1-palmitoyl-2-oléoyl-sn-phosphatidylcholine (POPC), le 1,2-distéaroyl-sn-glycéro-3-phospho-(1'-rac-glycérol) (DSPG), le 11,2-dioléoyl-sn-glycéro-3-phospho-(1'-rac-glycérol) (DOPG), la 1,2-distéaroyl-sn-glycéro-3-phosphocholine (DSPC ), la 1,2-dipalmitoyl-sn-glycéro-3-phosphocholine (DPPC), la 1,2-di-(9Z-octadécénoyl)-sn-glycéro-3-phosphoéthanolamine (DOPE) ou la 1,2-dihexadécanoyl-sn-glycéro-3-phosphoéthanolamine (DPPE) .

**Figure 1**

# Figure 2

**Figure 3**

**Figure 4**

**Figure 5**

Figure 5

# Figure 6

Figure 7

**A**

**B**

# Figure 8

**Figure 9**

Figure 10

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62666489
- US 8294007 B
- US 20150152494
- US 7504505 B
- US 5432272 A, Benner
- US 3687808 A, Merigan
- EP 1072679 A
- WO 9712896 A
- US 4845205 A
- US 5130302 A
- US 5134066 A
- US 5175273 A
- US 5367066 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5645985 A
- US 5830653 A
- US 5763588 A
- US 6005096 A
- US 5750692 A
- US 5681941 A
- US 7223833 B

**Non-patent literature cited in the description**

- **HOWORKA et al.** *Chemical Society reviews,* 2009, vol. 38, 2360-2384
- **KASIANOWICZ et al.** *Biochimica et. biophysica acta,* 2016, vol. 1858, 593-606
- **KOCER et al.** *Biosensors & bioelectronics,* 2012, vol. 38, 1-10
- **KUDR et al.** *Electrophoresis,* 2015, vol. 36, 2367-2379
- **LIU et al.** *International ed. in. English,* 2016, vol. 55, 15216-15222
- **LIU et al.** *Mikrochimica acta,* 2016, vol. 183, 955-963
- **OUKHALED et al.** *ACS chemical biology,* 2012, vol. 7, 1935-1949
- **SCHMIDT.** *Current opinion in. biotechnology,* 2016, vol. 39, 17-27
- **SHI et al.** *Analytical chemistry,* 2017, vol. 89, 157-188
- **YANG et al.** *Frontiers in. microbiology,* 2016, vol. 7, 1500
- **LIU et al.** *Chemical Society reviews,* 2012, vol. 41, 2283-2307
- **FAHIE et al.** *Analytical chemistry,* 2015, vol. 87, 11143-11149
- **GRENINGER et al.** *Genome medicine,* 2015, vol. 7, 99
- **GU et al.** Nanopore Single-Molecule Detection of Circulating microRNAs. *Clifton, N. J.,* 2013, vol. 1024, 255-268
- **KUKWIKILA et al.** *Analytical chemistry,* 2015, vol. 87, 9149-9154
- **WANG et al.** *ACS nano,* 2013, vol. 7, 9814-9822
- **WANG et al.** *Nature nanotechnology,* 2011, vol. 6, 668-674
- **MEIER et al.** *Physical Chemistry Chemical Physics (Incorporating Faraday Transactions),* 2000, vol. 2, 4559-4562
- **OSHIMA et al.** *Analytical chemistry,* 2013, vol. 85, 4363-4369
- **HIRANO-IWATA et al.** the ACS journal of surfaces and colloids. Langmuir, 2010, vol. 26, 1949-1952
- Ion Channel Reconstitution. Plenum Press, 1986
- **WHITE et al.** *Journal of the American Chemical Society,* 2007, vol. 129, 11766-11775
- **BAKER et al.** *Analytical chemistry,* 2013, vol. 85, 9078-9086
- **BRIGHT et al.** *ACS applied materials & interfaces,* 2013, vol. 5, 11918-11926
- **WATKINS et al.** the ACS journal of surfaces and colloids. Langmuir, 2011, vol. 27, 13618-13628
- **MALMSTADT et al.** *Advanced materials,* 2008, vol. 20, 84-89
- **ANDERSSON.** *Tethered and Polymer Supported Bilayer Lipid Membranes: Structure and Function,* 2016

- **JEON et al.** *Journal of the American Chemical Society,* 2006, vol. 128, 42-43
- **BRIGHT et al.** *ACS biomaterials science & engineering,* 2015, vol. 1, 955-963
- **SHENOY et al.** *Nano letters,* 2005, vol. 5, 1181-1185
- **HERON et al.** *J. Am. Chem. Soc.,* 2007, vol. 129, 16042-47
- **HILL et al.** *International review of cytology,* 1982, vol. 78, 1-125
- **HARTWIG et al.** *Protein Profile,* 1994, vol. 1, 706-778
- **HOLDEN et al.** *J. Am. Chem Soc.,* 2007, vol. 129, 8650-8655
- **LEPTIHN et al.** *Nature Protocols,* 2013, vol. 8
- **TONOOKA et al.** *Small,* 2014, vol. 10, 3275-3282
- **SZABO et al.** *Biochim. Biophys. Acta,* 2016, vol. 1858, 613-617
- **NORO et al.** *Soft Matter,* 2012, vol. 8, 6416-6429
- **LAFTAH et al.** *Polymer Hydrogels: A Review, Polymer-Plastics Technology and Engineering,* 2011, vol. 50, 1475-1486
- **COSTA et al.** *Chem. Soc. Rev.,* 2014, vol. 43, 3453-79
- **SUSAN M. FREIER ; KARL-HEINZ ALTMANN.** *Nucleic Acids Research,* vol. 25, 4429-4443
- **SANGHVI.** Antisense Research and Application. CRC Press, 1993
- **ENGLISCH et al.** *Angewandte Chemie, International Edition,* 1991, vol. 30, 613-722
- **CONCISE.** Encyclopedia of Polymer Science and Engineering. John Wiley & Sons, 1990, 858-859
- **COOK.** *Anti-Cancer Drug Design,* 1991, vol. 6, 585-607
- The Concise Encyclopedia Of Polymer Science And Engineering. John Wiley & Sons, 1990, 858-859
- **ENGLISCH et al.** *Angewandte Chemie, International Edition,* 1991, vol. 30, 613
- **SANGHVI, Y. S.** Antisense Research and Applications. CRC Press, 1993, 289-302
- **SAMBROOK et al.** Molecular Cloning: A Laboratory Manual. 1989
- Oligonucleotides and Analogues. Oxford University Press, 1991
- **KATZ.** *J. Am. Chem. Soc.,* 1951, vol. 74, 2238
- **YAMANE et al.** *J. Am. Chem. Soc.,* 1961, vol. 83, 2599
- **KOSTURKO et al.** *Biochemistry,* 1974, vol. 13, 3949
- **THOMAS.** *J. Am. Chem. Soc.,* 1954, vol. 76, 6032
- **ZHANG et al.** *J. Am. Chem. Soc.,* 2005, vol. 127, 74-75
- **ZIMMERMANN et al.** *J. Am. Chem. Soc.,* 2002, vol. 124, 13684-13685
- **ALBERTS ; JOHNSON ; LEWIS ; MORGAN ; RAFF ; ROBERTS ; WALTER ; WILSON ; HUNT.** Molecular Biology of the Cell. Garland, 2015
- Remington's Pharmaceutical Sciences. 1980
- **CHANDLER et al.** the ACS journal of surfaces and colloids. Langmuir, 2004, vol. 20, 898-905
- **MUELLER et al.** *Nature,* 1962, vol. 194, 979-980
- **BURDEN et al.** *J Phys Chem B,* 2000, vol. 104, 6103-6107
- **HIANIK et al.** Bilayer Lipid Membranes: Structure and Mechanical Properties. Kluwer Academic Publishers, 1995, 965
- **WOBSCHALL et al.** *Journal of Colloid and Interface Science,* 1971, vol. 36, 385-396
- **PASSECHNIK et al.** *Biofizika,* 1973, vol. 18, 655
- **NILES et al.** *Biophys. J.,* 1988, vol. 53, 327-335
- **WHITE et al.** *Biochem. Biophys. Acta,* 1973, vol. 323, 7-22
- **LANDAU et al.** *Theory of Elasticity,* 1965
- **WEISSTEIN.** Zone. *From MathWorld--A Wolfram Web Resource,* 09 October 2017, http://mathworld.wolfram.com/Zone.html
- **WOBSCHALL.** *Journal of Colloid and Interface Science,* 1971, vol. 36, 385-396
- **HIANIK, T. ; PASSECHNIK, V.** Bilayer Lipid Membranes: Structure and Mechanical Properties. Kluwer Academic Publishers, 1995
- **NÖDING et al.** *J. Phys. Chem.,* 2018, vol. 122 (16), 4537-4545
- **LE TRONG et al.** *Acta Crystallogr D Biol Crystallogr,* 2011, vol. 67, 813-821